# EUROPEAN PATENT SPECIFICATION

(11) **EP 1 631 575 B1**
(45) Date of publication and mention of the grant of the patent: **02.11.2006**
(21) Application number: 04732143.5
(22) Date of filing: 11.05.2004
(51) Int. Cl.: C07H 17/08, A61K 31/70, A61P 31/04

(54) **NEW 3-DECLADINOSYL 9a-N -CARBAMOYL AND 9a-N -THIOCARBAMOYL DERIVATIVES OF 9-DEOX-9-DIHYDRO-9a-AZA-9A-HOMOERYTHROMYCIN A**
NEUE 3-DECLADINOSYL-9A-N-CARBAMOYL- UND 9A-N-THIOCARBAMOYLDERIVATE VON 9-DEOXO-9-DIHYDRO-9A-AZA-9A-HOMOERYTHROMYCIN A
DERIVES DE 3-DECLADINOSYLE DE LA 9A-N-CARBAMOYL- ET DE LA 9A-N -THIOCARBAMOYL-9-DESOXO-9-DIHYDRO-9A-AZA-9A-HOMOERYTHROMYCINE A

(30) Priority: 14.05.2003 HR 20030381
(43) Date of publication of application: 08.03.2006
(73) Proprietor: GlaxoSmithKline istrazivacki centar Zagreb d.o.o., 10000 Zagreb (HR)
(72) Inventor: MARUSIC ISTUK, Zorica, 10 430 Samobor (HR); KUJUNDZIC, Nedjeljko, 10 000 Zagreb (HR); MUTAK, Stjepan, 10 000 Zagreb (HR)
(74) Representative: von Füner, Nicolai
(86) International application number: PCT/HR2004/000014
(87) International publication number: WO 2004/101591

(56) References cited:
- EP-A- 0 657 464
- WO-A-00/66603
- WO-A-02/12260
- WO-A-02/068438

## Description

### 1) Technical Field of the Invention:

A 61 K 31/70, C 0 7H 17/08

### 2) Technical Problem:

The invention relates to novel compounds from the class of azalide antibiotics. Particularly, the invention relates to novel 3-decladinosyl derivatives from the class of 9a-*N*-carbamoyl- and 9a-*N*-thiocarbamoyl-9-deoxo-9-dihydio-9a-aza-9a-homoerythromycin A, to their pharmaceutically acceptable addition salts with inorganic or organic acids, to their hydrates, to the process for their preparation, to the process for preparation of their pharmaceutical compositions and to the use thereof as antibiotics or intermediates for the synthesis of other macrolide antibiotics.

### 3) Prior Art:

Macrolides are well known agents for treating broad spectrum of infections. Erythromycin A (McGuire J. M., *Antibiot. Chemother*. 1952; 2: 281) has been for more than 40 years considered as safe and efficient agent for the treatment of respiratory and genital infections caused by Gram-positive and by some Gram-negative bacteria, some species of *Legionella, Mycoplasma, Chlamidia* and *Helicobacter.* By oximation of C-9 ketone of erythromycin and subsequent Beckmann rearrangement and reduction, 9-deoxo-9-dihydro-9a-aza-9a-homoerythromycin A, the first 15-membered macrolide antibiotics with 9a-amino group incorporated in aglycone ring, is obtained (Kobrehel G. et al., U.S. 4,328,334 5/1982).
By *O*-methylation of C-6 hydroxyl group of erythromycin clarithromycin is obtained (6-*O*-metil-erythromycin A) (Morimoto S. et al., *J. Antibiotics* 1984, 37, 187). In comparison with erythromycin A, clarithromycin is more stable and shows enhanced *in vitro* activity against Gram-positive strains (Kirst H. A. et al., *Antimicrob. Agents and Chemother.* 1989, 1419).
9a-*N*-carbamoyl and 9a-*N*-thiocarbamoyl derivatives of 9-deoxo-9-dihydro-9a-aza-9a-homoerythromycin A, that showed significant antibacterial activity, are described. (Kujundi 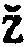 ć N. et. al., *Eur. J. Med. Chem*. 1995, 30, 455).
It is known as well that recent research on 14-membered macrolides has lead to the discovery of new classes of macrolide antibiotics, ketolides and anhydrolides. Instead of the neutral sugar L-cladinose known for its instability even in a weak acidic medium, ketolides possess a keto group on C-3 position (Agouridas C. et al., EP 596802 Al 5/1994, Le Martret O., FR 2697524 A1 5/94). Anhydrolides are characterized by 2,3-anhydro group (Elliott R. et al., *J. Med Chem.* 1998, 41, 1651). Ketolides show a significantly better activity against MLS (macrolide, lincosamide and streptogramin B) induced-resistant organisms (Jamjian C., *Antimicrob. Agents Chemother.* 1997, 41, 485).
Object of the present invention are 3-decladinosyl derivatives of 9a-*N*-carbamoyl- and 9a-*N*-thiocarbamoyl-9-deoxo-9-dihydro-9a-aza-9a-homoerythromycin A, their pharmaceutically acceptable addition salts with inorganic or organic acids, their hydrates, methods and intermediates for their preparation as well as preparation and application methods of pharmaceutical preparations.

### 4) Description of technical problem with examples

The invention relates to:
i) novel 3-decladinosyl derivatives of 9a-*N*-carbamoyl- and 9a-*N-*thiocarbamoyl-9-deoxo-9-dihydro-9a-aza-9a-homoerythromycin A, their pharmaceutically acceptable addition salts with inorganic or organic acids and their hydrates,
ii) methods for preparation of novel 3-decladinosyl derivatives of 9a-*N-*carbamoyl- and 9a-*N*-thiocarbamoyl-9-deoxo-9-dihydro-9a-aza-9a-homoerythromycin A, their pharmaceutically acceptable addition salts with inorganic or organic acids and their hydrates,
iii) use of novel 3-decladinosyl derivatives of 9a-*N*-carbamoyl- and 9a-*N-*thiocarbamoyl-9-deoxo-9-dihydro-9a-aza-9a-homoerythromycin A, their pharmaceutically acceptable addition salts with inorganic or organic acids and their hydrates as antibiotics or
iv) use of novel 3-decladinosyl derivatives of 9a-*N*-carbamoyl- and 9a-*N-*thiocarbamoyl-9-deoxo-9-dihydro-9a-aza-9a-homoerythromycin A, their pharmaceutically acceptable addition salts with inorganic or organic acids and their hydrates as intermediates for the synthesis of other macrolide antibiotics.

Novel 3-decladinosyl derivatives of 9a-*N*-carbamoyl- and 9a-*N*-thiocarbamoyl-9-deoxo-9-dihydro-9a-aza-9a-homoerythromycin A of the general formula (**I**), 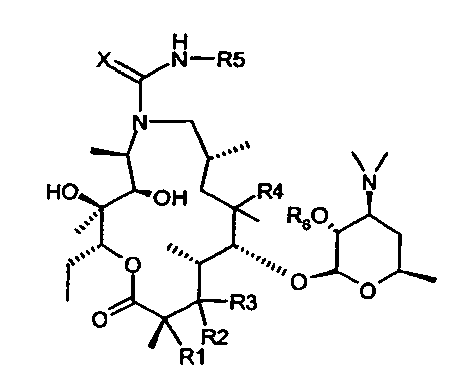 their pharmaceutically acceptable addition salts with inorganic or organic acids and their hydrates, wherein
R₁ individually stands for hydrogen or together with R₂ stands for double bond,
R₂ individually stands for hydrogen, hydroxyl or a group of the formula (II), 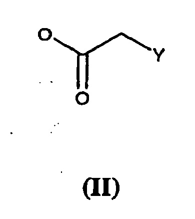 wherein
   Y individually stands for monocyclic aromatic ring unsubstituted or substituted with groups which are selected independently from halogen, OH, OCH₃, NO₂, NH₂
      or
   R₂ together with R₃ stands for ketone or together with R₁ stands for double bond,
   R₃ individually stands for hydrogen or together with R₂ stands for ketone or together with R₄ stands for ether,
   R₄ individually stands for hydroxyl, OCH₃ group or together with R₃ stands for ether,
   R₅ stands for C₁-C₄ alkyl group, C₂-C₄ alkenyl group, -(CH₂)ₘ-Ar, wherein Ar individually stands for phenyl or phenyl substituted with one or two groups which are selected independently from halogen or halogen alkyl, and m is 0-3,
   R₆ individually stands for hydrogen or hydroxyl protecting group and
   X stands for oxygen or sulphur
   are subject of this invention.

Term «hydroxyl protecting group« includes, but is not limited to benzoyl, benzyloxycarbonyl, acetyl or substituted silyl group in order to block the undesired reaction during the synthesis (Green T. H. and Wuts P. G. M., Protective Groups in Organic Synthesis, 3rd edition, John Wiley & Sons, New York, 1999).

Compounds given by general formula (I), wherein R₁, R₂, R₃, R₄, R₅, R₆, X and Y have the meaning as defined above could be prepared by methods described in this invention. Methods of preparations, which also are subject of this invention, are illustrated by schemes 1. and 2.:

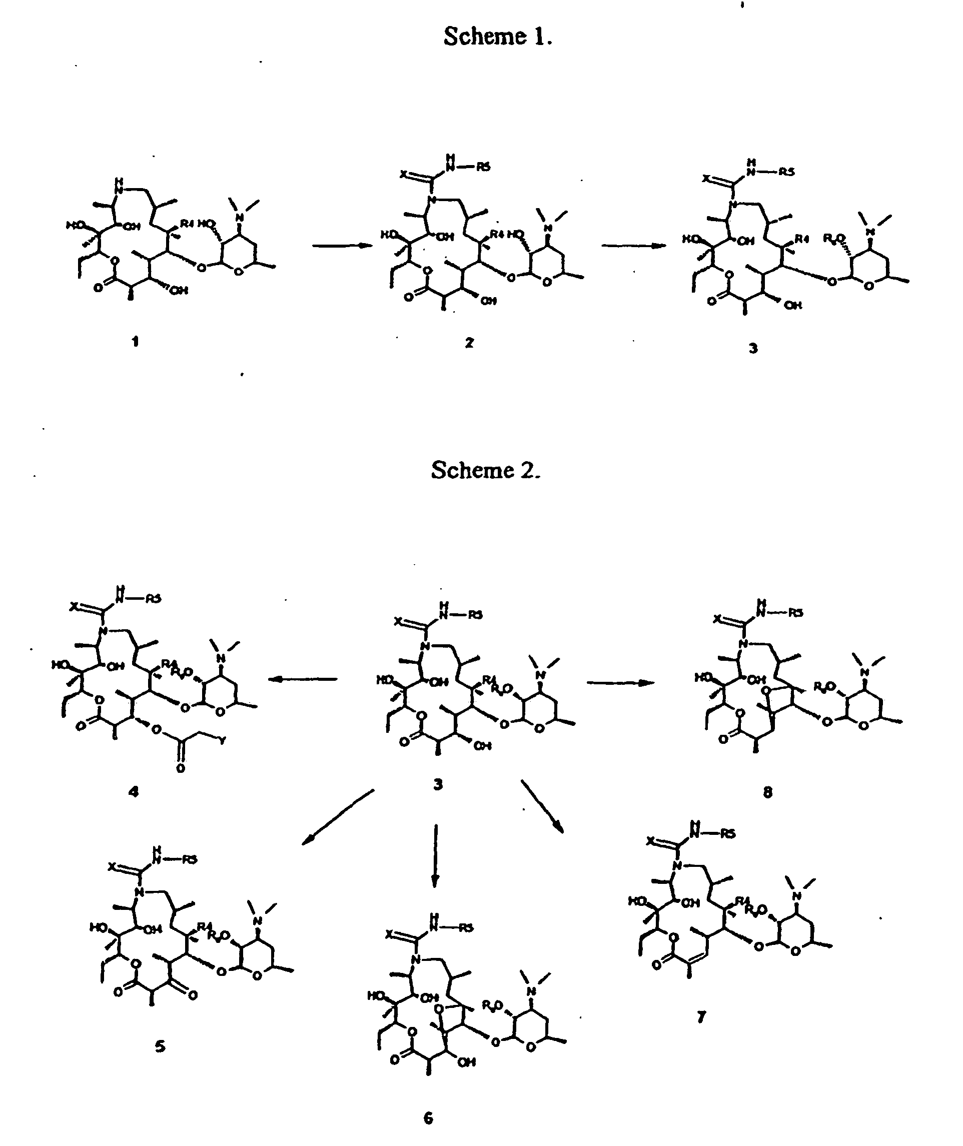

Starting compound described in scheme 1. for synthesis of compounds, which are subject of this invention are prepared by methods described in patent Kobrehel G. et al.,U.S. 4,328,334 5/1982 and in the article Denis A. and Agouridas C., *Bioorg. Med. Chem. Lett.* 1998, S, 2427 (compound of general formula 1).

### Step 1.

Compounds of the formula **1.**, wherein R₄ individually stands for hydroxyl or OCH₃ group are subjected to a reaction with isocyanate or isothiocyanate of the formula R₅₋N=C=X wherein R₅ and X have the above meanings, in a reaction-inert solvent preferably toluene or acetonitril, at a temperature from room temperature to the refluX temperature of the solvent for 30 min to 50 hours, yielding 9a-*N*-carbamoyl and 9a-*N-*thiocarbamoyl derivatives of the formula **2**. (scheme 1.), wherein R₄ individually stands for hydroxyl or OCH₃ group and R₅ and X have the above meanings.

9a-N-carbamoyl and 9a-*N*-thiocarbamoyl derivatives of the formula 2 wherein all substituents have the above meanings are subjected to a selective acylation of the hydroxyl group on 2'-position. Acylation is carried out with chlorides or anhydrides of carboxylic acids with up to 4 carbon atoms, preferably with acetic acid :anhydride, in the presence of inorganic or organic base, in a reaction-inert solvent at a temperature from 0-30°C, yielding 2'-O-acyl derivatives of the formula **3** (scheme 1.), wherein R₄ individually stands for hydroxyl or OCH₃ group, R₅ and X have the above meanings and R₆ stands for hydroxyl protecting group, preferably acetyl.

As suitable bases sodium hydrogencarbonate, sodium carbonate, potassium carbonate, triethylamine, pyridine, tributylamine are used. As a suitable inert solvent methylene chloride, dichlorethane, acetone, pyridine, ethyl acetate, tetrahydrofuran are used.

### Step 2.

2'-O-Acetyl derivatives from the Step 1. are optionally subjected to a reaction with mixed anhydrides of carboxylic acids of the formula Z-COO-R', wherein Z individually stands for hydrogen or for group Y, which is defined above, R' stands for the group which is usually used for preparation of mixed anhydrides as pivaloyl-, p-toluensulphonyl-, isobutoxycarbonyl-, etoxycarbonyl- or isopropoxycarbonyl-group, in the presence of inorganic or organic base, in a reaction-inert solvent, preferably methylene chloride, at a temperature from 0-30°C for 3-100 hours, yielding compounds of the formula **4**. (scheme 2.), wherein R₄ individually stands for hydroxyl or OCH₃ group, R₆ stands for acetyl and substituents R₅, X and Y have the above meanings. Formed compounds are subsequently subjected to deprotection with lower alcohols, preferably in methanol, at a temperature from room temperature to the reflux temperature of the solvent, yielding a compound of the formula **4**. (scheme 2.), wherein R₆ stands for hydrogen and R₄, R₅, X and Y have the above meanings,
or optionally
2'-O-acetyl derivatives from the Step 1., wherein R₄ stands for the OCH₃ group and all other substituents have the meanings as in the Step 1. are subjected to oxidation of the hydroxyl group in the C-3 position of an aglycone ring according to a modified Moffat-Pfitzner process with N,N-dimethylaminopropyl-3-ethyl-carbodiimide in the presence of dimethylsulfoxide and pyridinium trifluoracetate as a catalyst in an inert organic solvent, preferably in methylene chloride, at a temperature from 10 °C to room temperature, yielding compounds of the formula 5. (scheme 2.), wherein R₄ stands for the OCH₃ group, R₆ stands for acetyl and supstituents R₅ and X have the above meanings. Formed compounds are subsequently subjected to deprotection with lower alcohols, preferably in methanol, at a temperature from room temperature to the reflux temperature of the solvent, yielding a compound of the formula **5**. (scheme 2.), wherein R₆ stands for hydrogen and all other supstituents have the above meanings. Alternatively it is possible to oxidize C-3 hydroxyl group using Dess Martin periodinane reagens
or optionally 2'-O-acetyl derivatives from the Step 1., wherein R₄ stands for hydroxyl and all other supstituents have the meanings as in the Step 1, are subjected to oxidation described to obtain compounds of the formula 5. (scheme 2.), to obtain compounds with 3,6-hemiketal structure given by formula **6**. (scheme 2.), wherein R₆ stands for acetyl and R₅ and X have the above meanings are formed. Formed compounds are subsequently subjected to deprotection with lower alcohols, preferably in methanol, at a temperature from room temperature to the reflux temperature of the solvent, yielding a compound of the formula **6**. (scheme 2.), wherein R₆ stands for hydrogen and R₅ and X have the above meanings
or optionally
2'-O-acetyl derivatives from the Step 1., wherein R₄ stands for the OCH₃ group and all other substituents have the meanings as in the Step 1., are subjected to the adequate reagents for dehydratation, preferably methylsulfonyl anhydride to transform hydroxyl group on position 3 in good leaving group, in an inert organic solvent, preferably in pyridine, at a temperature from room temperature to the reflux temperature of the solvent for 10-50 hours. Formed intermediate is subsequently subjected to reaction of elimination with adequate reagens, preferably sodium hydride, in an inert organic solvent, preferably in tetrahydrofuran, at a temperature from 10 °C to room temperature, yielding 2,3-anhydro derivatives of the formula **7.** (scheme 2.), R₄ stands for the OCH₃ group, R₆ stands for acetyl and R₅ and X have the above meanings. Formed compounds are subsequently subjected to deprotection with lower alcohols, preferably in methanol, at a temperature from room temperature to the reflux temperature of the solvent, yielding a compound of the formula **7**. (scheme 2.), wherein R₆ stands for hydrogen and R₄, R₅ and X have the above meanings.
or optionally
2'-O-acetyl derivatives from the Step 1., wherein R₄ stands for the OH group and all other substituents have the meanings as in the Step 1., are subjected to adequate reagents for dehydratation, described to obtain compounds of the formula **7**. (scheme 2.), yielding 3,6 cyclic ethers of the formula **8**. (scheme 2.), wherein R₆ stands for acetyl and R₅ and X have the above meanings. Formed compounds are subsequently subjected to deprotection with lower alcohols, preferably in methanol, at a temperature from room temperature to the reflux temperature of the solvent, yielding a compound of the formula **8**. (scheme 2.), wherein R₆ stands for hydrogen and R₅ and X have the above meanings.

Pharmaceutically acceptable addition salts which are also subject of this invention are prepared by reaction of new compounds of the general formula **(I)** with at least one eqimolar amount of suitable inorganic or organic acid as chloride, iodide, sulphate, phosphate, acetic, propionic, trifluoracetic, maleinic, citric, stearic, jantaric, ethyljantaric, mathansulphonic, p-toluensulphonic, laurylsulphonic and other acids in reaction inert solvent. Addition salts are isolated by filtration (if they are insoluble in used solvent), by precipitation, by evaporating the solvent or by liophilisation.

The process is illustrated by the following examples, which do not limit the scope of the invention in any way.

### Example 1.

### 3-Decladinosyl-9-deoxo-9-dihydro-9a-N-(N'-isopropylcarbarnoyl)-9a-aza-9a-homoerythromycin A

To a solution of 3-decladinosyl-9-deoxo-9-dihydro-9a-aza-9a-homoerythromycin A (1,00 g) in toluene (20 ml) isopropylisocyanate (0,17 g) was added. Reaction mixture was stirred for 1 hour at room temperature. The solvent was evaporated and 1,15 g of crude product is obtained.
Crystallization from mixture acetone-petrol ether yielding 0,97 g of chromatographically homogenous title product with following physical-chemical constants:
- **TLC**: R_{f}= 0,37 (CH₂Cl₂ : MeOH= 8 : 2)
- **IR (KBr) [ν/cm**^{**-1**}**]:**: 3498, 3466, 2974, 2938, 2877, 1726, 1618, 1596, 1528, 1458, 1373, 1279, 1172, 1111, 1082, 1036, 978, 956, 932, 901, 833, 776,689,633.
- **MS m/z:**: **(FAB):** MH⁺ = 662
^{**1**}**H NMR (300 MHz, CDCl**_{**3**}**) [δ/ppm]**
5.03 (13-H), 4.40 (1'-H), 4.37 (9a-NCON'H), 3.92 (1"-H), 3.89 (3-H), 3.50 (11-H), 3.69 (5'-H), 3.63 (5-H), 3.32 (2'-H), 2.60 (2-H), 2.54 (3'-H), 2.28 (3'-N(CH₃)₂), 1.88 (14-Ha), 1.72 (4'-Ha), 1.58 (14-Hb), 1.56 (4-H), 1.34 (4' -Hb), 1.29 (2-CH₃), 1.27 (5'-CH₃), 1.26 (10-CH₃), 1.13 (1"-(CH₃)₂), 1.10 (12-CH₃), 1.06 (8-CH₃), 0.93 (4-CH₃), 0.89 (15-CH₃).
^{**13**}**C NMR (75 MHz, CDCl**_{**3**}**) [δ/ppm]**
177.3 (1-C), 158.8 (9a-NCONH), 105.8 (1'-C), 95.9 (5-C), 77.7 (13-C), 77.1 (12-C), 74.5 (11-C), 74.9 (3-C), 74.2 (6-C), 73.0 (9-C), 70.3 (5'-C), 69.2 (2'-C), 64.9 (3'-C), 44.6 (2-C), 42.4 (1 "-C), 41.7 (7-C), 39.9 (3'-N(CH₃)₂), 38.5 (4-C), 27.6 (4'-C), 25.8 (6-CH₃), 23.1 (1"-(CH₃)₂), 21.2 (14-C), 20.6 (5'-CH₃), 19.4 (8-CH₃), 16.6 (12-CH₃), 15.9 (2-CH₃), 12.2 (10-CH₃), 10.0 (15-CH₃), 7.7 (4-CH₃).

### Example 2.

### 3-Decladinosyl-9-deoxo-9-dihydro-9a-N-(N'-ethylcarhamoyl)-9a-aza-9a-homoerythromycin A

To a solution of 3-decladinosyl-9-deoxo-9-dihydro-9a-aza-9a-homoerythromycin A (1,00 g) in toluene (20 ml) ethylisocyanate (0,175 g) was added. Reaction mixture was stirred for 1 hour at room temperature. The solvent was evaporated and 1,24 g of crude product is obtained.
Crystallization from mixture acetone-petrol ether yielding 0,98 g of chromatographically homogenous title product with following physical-chemical constants:
- **TLC**: R_{f}= 0,48 (CH₂Cl₂ : MeOH = 8 : 2)
- **IR (KBr) [ν/cm**^{**-1**}**]:**: 3433, 2975, 2936, 2878,1730,1618,1532, 1458, 1382, 1350, 1267,1172,1111,1077,1036,980,956,933,900,834,764.
- **MS m/z:**: **(FAB):** MH⁺ = 648
^{**1**}**H NMR (300 MHz, CDCl**_{**3**}**) [δ/ppm]**
5.03 (13-H), 4.39 (1'-H), 4.56 (9a-NCON'H), 3.86 (3-H), 3.57 (11-H), 3.68 (5'-H), 3.62 (5-H), 3.43 (9-Ha), 3.31 (2'-H), 3.23 (N'CH₂), 2.59 (2-H), 2.53 (3'-H), 2.47 (9-Hb), 2.27 (3'-N(CH₃)₂), 1.90 (14-Ha), 1.71 (4'-Ha), 1.55 (14-Hb), 1.55 (4-H), 1.33 (4'-Hb), 1.28 (2-CH₃), 1.28 (15-CH₃), 1.28 (10-CH₃), 1.27 (5'-CH₃), 1.11 (N'CH₂CH₃), 1.08 (12-CH₃), 1.05 (8-CH₃), 0.92 (4-CH₃), 0.87 (15-CH₃).
^{**13**}**C NMR (75 MHz, CDCl**_{**3**}**) [δ/ppm]**
177.0 (1-C), 159.5 (9a-NCONH), 105.7 (1'-C), 96.2 (5-C), 77.7 (13-C), 77.1 (12-C), 74.5 (11-C), 75.1 (3-C), 74.1 (6-C), 72.8 (9-C), 70.3 (5'-C), 69.2 (2'-C), 64.9 (3'-C), 44.7 (2-C), 41.1 (7-C), 39.9 [3'N-(CH₃)₂], 38.3 (4-C), 36.5 (N'CH₂), 28.9 (8-C), 27.6 (4'-C), 25.6 (6-CH₃), 21.3 (14-C), 20.6 (5'-CH₃), 19.3 (8-CH₃), 16.6 (12-CH₃), 15.8 (2-CH₃), 15.0 (N'CH₂CH₃), 12.1 (10-CH₃), 10.3 (15-CH₃), 7.6 (4-CH₃).

### Example 3.

### 3-Decladinosyl-9-deoxo-9-dihydro-9a-N-[N'-(t-buthyl)carbamoyl]-9a-aza-9a-homoerythromycin A

To a solution of 3-decladinosyl-9-deoxo-9-dihydro-9a-aza-9a-homoerythromycin A (1,00 g) in toluene (20 ml) t-buthylisocyanate (0,198 g) was added. Reaction mixture was stirred for 1 hour at room temperature. The solvent was evaporated and 1,20 g of crude product is obtained.
Crystallization from mixture acetone-petrol ether yielding 0,95 g of chromatographically homogenous title product with following physical-chemical constants:
- **TLC**: R_{f}= 0,42 (CH₂Cl₂ : MeOH = 8 : 2)
- **IR (KBr) [v/cm**^{**-1**}**]:**: 3497, 2976, 2946, 1725, 1626, 1532, 1456, 1364, 1346, 1317, 1281, 1175, 1112, 1082, 1055, 1034, 977, 956, 931, 901, 865, 776, 685, 634.
- **MS m/z:**: (FAB): MH⁺ = 676
^{**1**}**H NMR (300 MHz, CDCl**_{**3**}**) [δ/ppm]**
5.03 (13-H), 4.52 (9a-NCON'H), 4.41 (1'-H), 3.89 (3-H), 3.68 (5'-H), 3.47 (11-H), 3.62 (5-H), 3.41 (9-Ha), 3.32 (2'-H), 2.59 (2-H), 2.54 (3'-H), 2.28 (3'-N(CH₃)₂), 1.88 (14-Ha), 1.72 (4'-Ha), 1.57 (14-Hb), 1.56 (4-H), 1.32 (4'-Hb), 1. 31 (N'C(CH₃)₃), 1.30 (2-CH₃), 1.28 (5'-CH₃), 1.24 (10-CH₃), 1.11 (12-CH₃), 1.05 (8-CH₃), 0.92 (4-CH₃), 0.88 (15-CH₃),.
^{**13**}**C NMR (75 MHz, CDCl**_{**3**}**) [δ/ppm]**
177.7 (1-C), 158.7 (9a-NCONH), 105.9 (1'-C), 95.7 (5-C), 77.9 (13-C), 77.1 (12-C), 74.6 (11-C), 74.8 (3-C), 74.3 (6-C), 73.3 (9-C), 70.3 (5'-C), 69.2 (2'-C), 65.0 (3'-C), 50.8 (N'C(CH₃)₃), 44.6 (2-C), 41.1 (7-C), 40.0 (3'N-(CH₃)₂), 38.6 (4-C), 29.1 (N'C(CH₃)₃), 28.9 (8-C), 27.7 (4'-C), 26.2 (6-CH₃), 21.2 (14-C), 20.6 (5'-CH₃), 19.4 (8-CH₃), 16.5 (12-CH₃), 16.0 (2-CH₃), 12.2 (10-CH₃), 10.6 (15-CH₃), 7.8 (4-CH₃).

### Example 4.

### 3-Decladinosyl-9-deoxo-9-dihydro-9a-N-(N'-benzylcarbamoyl)-9a-aza-9a-homoerythromycin A

To a solution of 3-decladinosyl-9-deoxo-9-dihydro-9a-aza-9a-homoerythromycin A (1,00 g) in toluene (20 ml) benzylisocyanate (0,25 g) was added. Reaction mixture was stirred for 1 hour at room temperature. Crude product (1.18 g) is obtained by filtration of precipitate from reaction mixture.
Crystallization from mixture acetone-petrol ether yielding 0,82 g of chromatographically homogenous title product with following physical-chemical constants:
- **TLC**: R_{f}= 0,42 (CH₂Cl₂ : MeOH = 8 : 2)
- **IR (KBr) [ν/cm**^{**-1**}**]:**: 3404, 2975, 2936, 1731, 1625, 1529, 1454, 1384, 1349, 1319, 1274, 1172, 1111, 1091, 1049, 978, 957, 931, 900, 865, 837, 740, 700, 635.
- **MS m/z:**: **(ES):** MH⁺ = 710,6
^{**1**}**H NMR (300 MHz, CDCl**_{**3**}**) [δ/ppm]**
7.35 (4"-H, 6"-H), 7.30 (3"-H, 7"-H), 7.24 (5"-H), 5.03 (13-H), 4.55 (1"-Ha), 4.48 (9a-NCON'H), 4.30 (1'-H), 4.30 (1"-Hb), 3.89 (3-H), 3.68 (5'-H), 3.51 (11-H), 3.62 (5-H), 3.41 (9-Ha), 3.30 (2'-H), 2.62 (2-H), 2.30 (3'-H), 2.27 (3'-N(CH₃)₂), 1.89 (14-Ha), 1.69 (4'-Ha), 1.58 (14-Hb), 1.56 (4-H), 1.28 (4'-Hb), 1.30 (2-CH₃), 1.27 (5'-CH₃), 1.32 (10-CH₃), 1.28 (6-CH₃), 1.10 (12-CH₃), 1.04(8-CH₃), 0.93 (4-CH₃), 0.89 (15-CH₃),.
^{**13**}**C NMR (75 MHz, CDCl**_{**3**}**) [δ/ppm]**
176.9 (1-C), 159.6 (9a-NCONH), 139.4 (2"-C), 128.6 (4"-C, 6"-C), 127.3 (3"-C, 7"-C), 126.9 (5"-C), 105.7 (1'-C), 96.4 (5-C), 77.7 (13-C), 77.1 (12-C), 75.0 (11-C), 75.0 (3-C), 74.2 (6-C), 74.4 (9-C), 70.2 (5'-C), 69.2 (2'-C), 64.8 (3'-C), 44.7 (2-C), 44.6 (1"-C), 44.6 (7-C), 40.0 (3'N-(CH₃)₂), 38.4 (4-C), 27.5 (4'-C), 25.3 (6-CH₃), 21.3 (14-C), 20.7 (5'-CH₃), 19.7 (8-CH₃), 16.7 (12-CH₃), 15.9 (2-CH₃), 12.2 (10-CH₃), 10.4 (15-CH₃), 7.6 (4-CH₃).

### Example 5.

### 3-Decladinosyl-9-deoxo-9-dihydro-9a-N-[N'-(3-trifluoroxnethylphenyl)carbamoyl]-9a-aza-9a-homoerythromycin A

To a solution of 3-decladinosyl-9-deoxo-9-dihydro-9a-aza-9a-homoerythromycin A (1,00 g) in toluene (20 ml) 3-trifluormethylphenylisocyanate (0,33 g) was added.
Reaction mixture was stirred for 30 min at room temperature. The solvent was evaporated and 1,30 g of crude product is obtained.
Crystallization from mixture ethyl ether -petrol ether yielding 0,98 g of chromatographically homogenous title product with following physical-chemical constants:
- **TLC**: R_{f}= 0,51 (CH₂Cl₂ : MeOH = 8 : 2)
- **IR (KBr) [ν/cm**^{**-1**}**]:**: 3451, 2975, 2935, 1727, 1704, 1659, 1548, 1494, 1449, 1384, 1336, 1258, 1167, 1125, 1072, 1049, 979, 957, 933, 901, 864, 835,794,758,699,659.
- **MS m/z:**: **(ES):** MH⁺ = 764,6
^{**1**}**H NMR (300 MHz, CDCl**_{**3**}**) [δ/ppm]**
7.70 (2"-H), 7.58 (6"-H), 7.35 (5"-H), 7.23 (4"-H), 4.82 (13-H), 4.62 (9a-NCON'H), 4.35 (1 '-H), 3.87 (3-H), 3.62 (5'-H), 3.62 (5-H), 3.51 (11-H), 3.41 (9-Ha), 3.30 (2'-H), 2.66 (2-H), 2.50 (3'-H), 2.26 (3'-N(CH₃)₂), 1.88 (14-Ha), 1.71 (4'-Ha), 1.58 (14-Hb), 1.56 (4-H), 1.28 (6-CH₃), 1.28 (2-CH₃), 1.31 (4'-Hb), 1.27 (5'-CH₃), 1.24 (10-CH₃), 1.10 (12-CH₃), 1.04 (8-CH₃), 0.92 (4-CH₃), 0.88 (15-CH₃).
^{**13**}**C NMR (75 MHz, CDCl**_{**3**}**) [δ/ppm]**
176.8 (1-C), 156.6 (9a-NCONH), 140.0 (1"-C), 131.3 (3"-C), 129.2 (5"-C), 122.3 (6"-C), 118.9 (4"-C), 115.8 (2"-C), 106.1 (1'-C), 96.5 (5-C), 78.8 (13-C), 77.1 (12-C), 75.7 (3-C), 74.8 (9-C), 74.7 (6-C), 74.6 (11-C), 70.6 (5'-C), 69.4 (2'-C), 65.2 (3'-C), 45.2 (2-C), 40.2 (3'N-(CH₃)₂), 38.8 (4-C), 27.9 (4'-C), 21.5 (14-C), 20.9 (5'-CH₃), 20.0 (8- CH₃), 16.8 (12-CH₃), 16.1 (2-CH₃), 13.8 (10-CH₃), 10.9 (15-CH₃), 7.7 (4-CH₃).

### Example 6.

### 3-Decladinosyl-9-deoxo-9-dihydro-9a-N-[N'-(2-trifluoroniethylphenyl)carbamoyl]-9a-aza-9a-homoerythromycin A

To a solution of 3-decladinosyl-9-deoxo-9-dihydro-9a-aza-9a-homoerythromycin A (1,00 g) in toluene (20 ml) 2-trifluormethylphenylisocyanate (0,35 g) was added.
Reaction mixture was stirred for 30 min at room temperature. The solvent was evaporated and 1,29 g of crude product is obtained.
Crystallization from mixture ethyl ether -petrol ether yielding 0,84 g of chromatographically homogenous title product with following physical-chemical constants:
- **TLC**: R_{f} = 0,69 (CH₂Cl₂ : MeOH = 8 : 2)
- **IR (KBr) [ν/cm**^{**-1**}**]:**: 3458,2975,2940,2879,2786, 1727,1658, 1591, 1536, 1457, 1384, 1322, 1282, 1244, 1171, 1112, 1035, 979, 956, 934, 901, 864, 834, 762, 702.
- **MS m/z:**: (FAB): MH⁺ = 764,3
^{**1**}**H NMR (300 MHz, CDCl**_{**3**}**) [δ/ppm]**
8.01 (6"-H), 7.55 (3"-H), 7.26 (4"-H), 7.16 (5"-H), 4.98 (13-H), 4.62 (9a-NCON'H), 4.38 (1'-H), 3.90 (3-H), 3.65 (5'-H), 3.64 (5-H), 3.61 (11-H), 3.31 (2'-H), 2.64 (2-H), 2.52 (3'-H), 2.27 (3'-N(CH₃)₂), 1.91 (14-Ha), 1.72 (4'-Ha), 1.57 (14-Hb), 1.56 (4-H), 1.35 (10-CH₃), 1.32 (2-CH₃), 1.28 (6-CH₃), 1.31 (4'-Hb), 1.24 (5'-CH₃), 1.15 (12-CH₃), 1.10 (8-CH₃), 0.96 (4-CH₃), 0.91 (15-CH₃).
^{**13**}**C NMR (75 MHz, CDCl**_{**3**}**) [δ/ppm]**
177.3 (1-C), 157.0 (9a-NCONH), 136.4 (1"-C), 132.5 (3"-C), 125.7 (5"-C), 125.1 (4"-C), 123.4 (6"-C), 105.9 (1'-C), 82.9 (5-C), 77.9 (13-C), 74.4 (12-C), 75.2 (3-C), 74.4 (6-C), 73.8 (11-C), 70.4 (5'-C), 69.2 (2'-C), 64.9 (3'-C), 44.7 (2-C), 39.9 (3'N-(CH₃)₂), 38.6 (4-C), 27.6 (4'-C), 21.3 (14-C), 20.7 (5'-CH₃), 19.1 (8-CH₃), 16.6 (12-CH₃), 15.9 (2-CH₃), 12.5 (10-CH₃), 10.5 (15-CH₃), 7.7 (4-CH₃).

### Example 7.

### 3-Decladinosyl-9-deoxo-9-dihydro-9a-N-[N'-(3-trifluoromethylphenyl)thiocarbamoyl]-9a-aza-9a-homoerythromycin A

To a solution of 3-decladinosyl-9-deoxo-9-dihydro-9a-aza-9a-homoerythromycin A (1,00 g) in toluene (20 ml) 3-trifluormethylphenylisocyanate (0,35 g) was added.
Reaction mixture was stirred for 15 min at room temperature. Crude product (1.18 g) is obtained by filtration of precipitate from reaction mixture.
Crystallization from mixture acetone-petrol ether yielding 0,92 g of chromatographically homogenous title product with following physical-chemical constants:
- **TLC**: R_{f}= 0,60 (CH₂Cl₂ : MeOH = 8 : 2)
- **IR (KBr) [ν/cm**^{**-1**}**]:**: 3449, 2975, 2937, 1708, 1600, 1547, 1494, 1452, 1384, 1328, 1255, 1164, 1116, 1073, 1019, 980, 956, 885, 862, 793, 735, 696.
- **MS m/z:**: (FAB): MH⁺ = 780
^{**1**}**H NMR (300 MHz, CDCl**_{**3**}**) [δ/ppm]**
7.68 (2"-H), 7.42 (6"-H), 7.26 (5"-H), 7.18 (4"-H), 4.82 (13-H), 4.61 (9a-NCON'H), 4.40 (1'-H), 3.90 (3-H), 3.66 (5'-H), 3.57 (5-H), 3.49 (11-H), 3.31 (2'-H), 2.69 (2-H), 2.52 (3'-H), 2.27 (3'-N(CH₃)₂), 1.95 (14-Ha),1.69 (4'-Ha), 1.62 (14-Hb), 1.53 (4-H), 1.34 (2-CH₃), 1.28 (6-CH₃), 1.34 (4'-Hb), 1.28 (5'-CH₃), 1.25 (10-CH₃), 1.21 (12-CH₃), 1.08 (8-CH₃), 0.98 (4-CH₃), 0.94 (15-CH₃).
^{**13**}**C NMR (75 MHz, CDCl**_{**3**}**) [δ/ppm]**
183.2 (9a-NCONH), 177.6 (1-C), 140.4 (1"-C), 130.5 (3"-C), 130.2 (5"-C), 128.1 (6"-C), 125.2 (4"-C), 123.6 (2"-C), 105.2 (1'-C), 94.9 (5-C), 79.3 (3-C), 77.7 (13-C), 77.2 (12-C), 73.5 (9-C), 73.6 (6-C), 73.4 (11-C), 70.6 (5'-C), 69.5 (2'-C), 65.2 (3'-C), 44.5 (2-C), 41.1 (7-C), 40.2 (3'N-(CH₃)₂), 38.8 (4-C), 28.1 (8-C), 27.9 (4'-C), 25.9 (6-CH₃), 21.0 (14-C), 21.0 (5'-CH₃), 18.9 (8-CH₃), 16.6 (12-CH₃), 16.1 (2-CH₃), 12.8 (10-CH₃), 10.8 (15-CH₃), 7.6 (4-CH₃).

### Example 8.

### 3-Decladinosyl-9-deoxo-9-dihydro-9a-N-(N'-benzylthiocarbamoyl)-9a-aza-9a-homoerythromycin A

To a solution of 3-decladinosyl-9-deoxo-9-dihydro-9a-aza-9a-homoerythromycin A (1,00 g) in toluene (20 ml) benzylisothiocyanate (0,38 g) was added. Reaction mixture was stirred for 1 hour at room temperature. The solvent was evaporated and 1,35 g of crude product is obtained.
Crystallization from mixture ethyl ether -petrol ether yielding 1,06 g of chromatographically homogenous title product with following physical-chemical constants:
- **TLC**: R_{f}= 0,40 (CH₂Cl₂ : MeOH = 8 : 2)
- **IR (KBr) [ν/cm**^{**-1**}**]:**: 3448, 2974, 2938, 1703, 1528, 1456, 1384, 1323, 1283, 1178, 1109, 1073, 1020, 978, 955, 934, 862, 825, 759, 699.
- **MS m/z:**: **(ES):** MH⁺ = 726.5
^{**1**}**H NMR (300 MHz, CDCl**_{**3**}**) [δ/ppm]**
7.34 (4"-H, 6"-H), 7.30 (3"-H, 7"-H), 7.27 (5"-H), 4.64 (9a-NCSN'H), 4.57 (13-H), 4.39 (1'-H), 3.89 (3-H), 3.67 (5'-H), 3.67 (5-H), 3.51 (11-H), 3.31 (2'-H), 2.64 (2-H), 2.55 (3'-H), 2.28 (3'-N(CH₃)₂), 1.93 (14-Ha), 1.73 (4'-Ha), 1.62 (14-Hb), 1.56 (4-H), 1.34 (2-CH₃), 1.27 (4'-Hb), 1.27 (5'-CH₃), 1.27 (10-CH₃), 1.25 (12-CH₃), 0.94 (8-CH₃), 0.87 (4-CH₃), 0.86 (15-CH₃).
^{**13**}**C NMR (75 MHz, CDCl**_{**3**}**) [δ/ppm]**
189.2 (9a-NCSNH), 179.5 (1-C), 137.5 (2"-C), 128.6 (4"-C, 6"-C), 128.2 (3"-C, 7"-C), 127.6 (5"-C), 105.9 (1'-C), 93.9 (5-C), 78.7 (13-C), 75.1 (12-C), 75.2 (6-C), 74.6 (3-C), 73.9 (11-C), 70.1 (5'-C), 69.5 (2'-C), 65.2 (3'-C), 50.8 (1"-C), 44.1 (2-C), 40.2 (3'N-(CH₃)₂), 44.4 (4-C), 27.9 (4'-C), 21.1 (14-C), 20.9 (5'-CH₃), 19.0 (8-Me), 16.7 (12-CH₃), 16.5 (2-CH₃), 12.3 (10-CH₃), 11.3 (15-CH₃), 8.3 (4-CH₃).

### Example 9.

### 3-Decladinosyl-9-deoxo-9-dihydro-9a-N-[N'-2,4-dichlorphenyl)carbamoyl]-9a-aza-9a-homoerythromycin A

To a solution of 3-decladinosyl-9-deoxo-9-dihydro-9a-aza-9a-homoerythromycin A (3,00 g) in toluene (50 ml) 2,4-dichlorphenylisocyanate (1,07 g) was added. Reaction mixture was stirred for 1 hour at room temperature. The solvent was evaporated and 3,80 g of crude product is obtained.
Crystallization from mixture ethyl ether -petrol ether yielding 4,26 g of chromatographically homogenous title product with following physical-chemical constants:
- **TLC**: R_{f}= 0,63 (CH₂Cl₂: MeOH = 8 : 2)
- **IR (KBr) [ν**/**cm**^{**-1**}**]:**: 3428, 2973, 2934, 2878, 1718,1659, 1580, 1517, 1459, 1382, 1345,1297,1229,1176,1110,1086,1048,978,955,932,901, 866,825,759,731,696,631.
- **MS m/z:**: **(ES):** MH⁺ = 764,5
^{**1**}**H NMR (300 MHz, CDCl**_{**3**}**) [δ/ppm]**
8.17 (6"-H), 7.33 (3"-H), 7.17 (5"-H), 4,71 (13-H), 4.57 (9a-NCON'H), 4.40 (1'-H), 4.10 (5-H), 3.89 (3-H), 3.89 (5'-H), 3.32 (2'-H), 2.62 (2-H), 2.60 (3'-H), 2.30 (3'-N(CH₃)₂), 1.93 (14-Ha), 1.76 (4'-Ha), 1.57 (14-Hb), 1.56 (4-H), 1.53 (12-CH₃), 1.36 (10-CH₃), 1.31 (2-CH₃), 1.29 (4'-Hb), 1.29 (8-CH₃), 1.27 (5'-CH₃), 0.94 (4-CH₃), 0.90 (15-CH₃).
^{**13**}**C NMR (75 MHz, CDCl**_{**3**}**) [δ/ppm]**
177.2 (1-C), 156.4 (9a-NCONH), 134.7 (1"-C), 128.4 (3"-C), 127.6 (2"-C), 125.3 (5"-C), 123.2 (4"-C), 105.9 (1'-C), 78.2 (13-C), 75.9 (3-C), 70.5 (5'-C), 69.5 (2'-C), 65.2 (3'-C), 44.9 (2-C), 40.2 (3'N-(CH₃)₂), 38.8 (4-C), 28.1 (4'-C), 28.0 (8-C), 21.5 (14-C), 20.9 (5'-CH₃), 19.4 (6-CH₃), 16.8 (12-CH₃), 16.2 (2-CH₃), 12.9 (10-CH₃), 10.9 (15-CH₃), 8.0 (4-CH₃).

### Example 10.

### 3-Decladinosyl-9-deoxo-9-dihydro-9a-N-[N'-allylthiocarbamoyl)-9a-aza-9a-homoerythromycin A

To a solution of 3-decladinosyl-9-deoxo-9-dihydro-9a-aza-9a-homoerythromycin A (1,00 g) in toluene (20 ml) allylisothiocyanate (0,19 g) was added. Reaction mixture was stirred for 1 hour at room temperature. The solvent was evaporated and 1,20 g of crude product is obtained.
Crystallization from mixture ethyl ether -petrol ether yielding 0,72 g of chromatographically homogenous title product with following physical-chemical constants:
- **TLC**: R_{f}= 0,68 (CH₂Cl₂ : MeOH = 8 : 2)
- **IR (KBr) [ν/cm**^{**-1**}**]:**: 3451, 2974, 2938, 2878, 1704, 1644, 1520, 1456, 1384, 1321, 1286, 1178, 1110, 1074, 1048, 1019, 978, 955, 934, 862, 833, 762, 632.
- **MS m/z:**: **(ES):** MH⁺ = 676.4
^{**1**}**H NMR (300 MHz, CDCl**_{**3**}**) [δ/ppm]**
5.94 (2"-H), 5.29 (3"-Ha), 5.20 (3"-Hb), 4.57 (13-H), 4.41 (1'-H), 4.37 (9a-NCSN'H), 4.34 (1"-Ha), 4.21 (1"-Hb), 3.90 (3-H), 3.69 (5'-H), 3.78 (5-H), 3.32 (2'-H), 2.64 (2-H), 2.54 (3'-H), 2.28 (3'-N(CH₃)₂), 1.92 (14-Ha), 1.70 (4'-Ha), 1.60 (14-Hb), 1.52 (4-H), 1.34 (4'-Hb), 1.35 (2-CH₃), 1.28 (5'-CH₃), 1.28 (6-CH₃), 1.26 (10-CH₃), 1.24 (12-CH₃), 1.12 (8-CH₃), 0.95 (4-CH₃), 0.94 (15-CH₃).
^{**13**}**C NMR (75 MHz, CDCl**_{**3**}**) [δ/ppm]**
189.3 (1-C), 179.7 (9a-NCSNH), 133.6 (2"-C), 117.8 (3"-C), 106.0 (1'-C), 95.0 (5-C), 78.6 (13-C), 75.1 (12-C), 75.1 (6-C), 74.8 (3-C), 73.8 (11-C), 70.4 (5'-C), 69.3 (2'-C), 65.4 (3'-C), 48.8 (1"-C), 44.2 (2-C), 39.9 (3'N-(CH₃)₂), 38.9 (4-C), 27.6 (4'-C), 20.7 (14-C), 20.6 (5'-CH₃), 18.7 (8-CH₃), 16.4 (12-CH₃), 16.2 (2-CH₃), 12.1 (10-CH₃), 10.9 (15-CH₃), 7.9 (4-CH₃).

### Example 11.

### 2'-O-Acetyl-3-decladinosyl-9-deoxo-9-dihydro-9a-N-(N'-isopropylcarbamoyl)-9a-aza-9a-homoerythromycin A

To a solution of 3-decladinosyl-9-deoxo-9-dihydro-9a-*N*-(*N*'-isopropylcarbamoyl)-9a-aza-9a-homoerythromycin A (1,00 g) in CH₂Cl₂ (20 ml), NaHCO₃ (1.01 g) and acetic acid anhydride (173µl) were added and it was then stirred for 24 hours at room temperature. Onto the reaction mixture a saturated NaHCO₃ solution was added, the layers were separated and the aqueous one was extracted two more times with CH₂Cl₂. The combined organic extracts were rinsed with saturated NaHCO₃ solution and water and evaporated yielding the title product (1,10 g) with following physical-chemical constants:
- **TLC**: R_{f} = 0.71 (CH₂Cl₂ : MeOH = 8 : 2).
- **IR (KBr) [νcm**^{**-1**}**]:**: 3423, 2974, 2938, 2878, 1735, 1624, 1560, 1522, 1459, 1376, 1252, 1169, 1109, 1058, 987, 956, 901, 834, 771, 670.
- **MS m/z:**: **(ES):** MH⁺ = 704,2

### Example 12.

### 2'-O-Acetyl-3-O-mesyl-3-decladinosyl-9-deoxo-9-dihydro-9a-N-(N'-isopropylcarbamoyl)-9a-aza-9a-homoerythromycin A

To a solution of 2'-*O*-Acetyl-3-decladinosyl-9-deoxo-9-dihydro-9a-*N*-(*N*'-isopropylcarbamoyl)-9a-aza-9a-homoerythromycin A (1,00 g) in pyridine (5 ml) methylsulphonyl anhydride (0,30 g) was added and the reaction mixture was stirred at room temperature for 3 hours. The solvent was evaporated and the residue was dissolved in CH₂Cl₂ (20 ml). Saturated aqueous solution of NaHCO₃ (20 ml) was added, the layers were separated and the water layer was extracted two more times with CH₂Cl₂ Combined organic extracts were rinsed with NaHCO₃ and brine, dried over K₂CO₃ and evaporated yielding 0,87 g of crude product with following physical-chemical constants
- **TLC**: R_{f}= 0,43 (CHCl₃ : MeOH : NH₄OH = 6 : 1 : 0.1).
- **IR (KBr) [νcm**^{**-1**}**]:**: 3422, 3065, 2972, 2876, 1735, 1637, 1618, 1535, 1487, 1459, 1377, 1330, 1240, 1206, 1193, 1059, 1004, 785, 773, 755, 682, 609.
- **MS m/z:**: (ES): MH⁺ = 783,2

### Example 13.

### 3-Decladinosyl-3,6-hemiketal-9-deoxo-9-dihydro-9a-N-(N'-isopropylcarbamoyl)-9a-aza-9a-homoerythromycin A

To a solution of 2'-*O*-Acetyl-3-decladinosyl-9-deoxo-9-dihydro-9a-*N*-(*N*'-isopropylcarbamoyl)-9a-aza-9a-homoerythromycin A (1,50 g) from Example 11. in CH₂Cl₂ (30 ml) dimethylsulfoxide (2,00 ml) and *N,N*-dimethyl-aminopropyl-ethyl-carbodiimid (2,30 ml) were added. The reaction mixture was cooled to 15 °C, and then, keeping the temperature constant, solution of pyridinium trifluoracetate (2,30 g) in CH₂Cl₂ (10 ml) was added dropwise during 30 minutes. The reaction mixture was stirred at 15 °C to room temperature for additional 2 hours. To the reaction mixture saturated aqueous solution of NaCl (40 ml) was added and the pH value was adjusted to pH 9.5. The layers were separated and the water layer was extracted two more times with CH₂Cl_{2.} Combined organic extracts were rinsed with brine, NaHCO₃ and water, dried over K₂CO₃ and evaporated yielding 1,36 g of the crude product which is dissolved in MeOH (50 ml) and stirred for 24 hours at room temperature. The solvent was evaporated and the residue purified by low pressure chromatography on a silica gel column using the system CH₂Cl₂ : MeOH : NH₄OH = 90 : 3 : 0,3. The combining and evaporating of chromatographically homogenous fractions gave the title product (0,313 g) with following physical-chemical constants:
- **TLC**: R_{f}= 0.51 (CH₂Cl₂ : MeOH : NH₄OH = 90 : 9: 0.5).
- **IR (KBr) [vcm**^{**-1**}**]:**: 3373, 2968, 2936, 2877,1728, 1716, 1615, 1526, 1520, 1456, 1384, 1261, 1181, 1099, 1031, 961, 864, 799, 737.
- **MS m/z:**: (ES): MH⁺ = 660,6
^{**1**}**H NMR (300 MHz, CDCl**_{**3**}**) [δ/ppm]**
4.91 (13-H), 4.21 (1'-H), 4.20 (9a-NCON'H), 3.96 (1"-H), 3.63 (11-H), 3.53 (5'-H), 3.78 (5-H), 3.26 (2'-H), 2.64 (2-H), 2.59 (10-H), 2.54 (3'-H), 2.32 (3'-N(CH₃)₂), 2.08 (4-H), 2.06 (8-H), 1.82 (14-Ha), 1.73 (4'-Ha), 1.62 (14-Hb), 1.48 (7-Ha), 1.39 (6-CH₃), 1.36 (7-Hb), 1.28 (10-CH₃), 1.27 (4'-Hb), 1.24 (5'-CH₃), 1.24 (4-CH₃), 1.15 (12-CH₃), 1.13 (1"-(CH₃)₂), 0.97 (8-CH₃), 0.96 (2-CH₃), 0.87 (15-CH₃).
^{**13**}**C NMR (75 MHz, CDCl**_{**3**}**) [δ/ppm]**
176.4 (1-C), 157.4 (9a-NCONH), 103.7 (1'-C), 103.1 (3-C), 94.2 (5-C), 83.8 (6-C), 83.3 (11-C), 79.0 (13-C), 75.4 (12-C), 69.7 (5'-C), 69.6 (2'-C), 65.4 (3'-C), 49.2 (2-C), 48.3 (4-C), 46.5 (10-C), 42.6 (1"-C), 41.4 (7-C), 40.4 (3'-N(CH₃)₂), 29.0 (4'-C), 28.8 (8-C), 26.3 (6-CH₃), 23.6 (1"-(CH₃)₂), 21.6 (14-C), 21.2 (5'-CH₃), 21.2 (8-CH₃), 17.7 (12-CH₃), 14.3 (10-CH₃), 13.9 (2-CH₃), 12.9 (4-CH₃), 10.8 (15-CH₃),.

### Example 14.

### 3-Decladinosyl-3,6-cyclic ether-9-deoxo-9-dihydro-9a-N-(N'-isopropylcarbamoyl)-9a-aza-9a-homoerythromycin A

To a solution of 2'-*O*-Acetyl-3-O-mesyl-3-decladinosyl-9-deoxo-9-dihydro-9a-*N*-(*N*'-isopropylcarbamoyl)-9a-aza-9a-homoerythromycin A from Example 12. (0,80 g) in DMF/THF (3,5 ml / 1 ml) suspension (60%) of NaH in mineral oil (163 mg) was added and the reaction mixture was stirred at 0 °C for 5 hours, and additional 20h at the room temperature. The reaction mixture was poured into saturated aqueos solution of NaHCO₃ (20 ml), EtOAc (20 ml) was added and the layers were separated. The water layer was extracted two more times with EtOAc. Combined organic extracts were rinsed with NaHCO₃ and brine, dried over K₂CO₃ and evaporated yielding 0.53 g of product. The obtained product was dissolved in MeOH (20 ml) was stirred for 24 hours at room temperature. The solvent was evaporated and the crude product was purified by chromatography on a silica gel column using the system CH₂Cl₂ : MeOH : NH₄OH = 90 : 3 : 0.3. The combining and evaporating of chromatographically homogenous fractions gave the title product (0.17 g) with following physical-chemical constants:
- **TLC**: R_{f}= 0.32 (CH₂Cl₂ : MeOH : NH₄OH = 90 : 9 : 0.5).
- **IR (KBr) [νcm**^{**-1**}**]:**: 3428, 2972, 2938, 2876, 1735, 1619, 1528, 1459, 1383, 1330, 1270, 1179, 1144, 1073, 1051, 1030, 961, 893, 535, 799, 759, 635.
- **MS m/z:**: (ES): MH⁺ = 644,2
^{**1**}**H NMR (300 MHz, CDCl**_{**3**}**) [δ/ppm]**
4.90 (13-H), 4.24 (1'-H), 4.20 (9a-NCON'H), 3.95 (1"-H), 3.83 (11-H), 3.64 (3-H), 3.55 (5'-H), 3.57 (5-H), 3.33 (2'-H), 3.25 (9-Ha), 2.92 (9-Hb), 2.76 (3'-H), 2.57 (2-H); 2.45 (3'-N(CH₃)₂), 2.06 (8-H), 2.04 (4-H), 1.92 (4'-Ha), 1.83 (14-Ha), 1.64 (14-Hb), 1.64 (7-Ha), 1.33 (4' -Hb), 1.29 (7-Hb), 1.28 (10-CH₃), 1.26 (5'-CH₃), 1.24 (4-CH₃), 1.23 (6-CH₃), 1.19 (2-CH₃), 1.14 (12-CH₃), 1.13 (1"-(CH₃)₂), .0.96 (8-CH₃), 0.88 (15-CH₃).
^{**13**}**C NMR (75 MHz, CDCl**_{**3**}**) [δ/ppm]**
176.6 (1-C), 157.8 (9a-NCONH), 103.9 (1'-C), 93.4 (5-C), 84.0 (6-C), 83.6 (3-C), 78.7 (13-C), 75.5 (12-C), 69.5 (2'-C), 69.4 (5'-C), 65.4 (3'-C), 46.5 (2-C), 45.7 (4-C), 42.6 (1"-C), 40.5 (3'-N(CH₃)₂), 40.3 (7-C), 30.1 (4'-C), 28.9 (8-C), 23.6 (1"-(CH₃)₂), 23.1 (6-CH₃), 21.7 (14-C), 21.3 (8-CH₃), 21.1 (5'-CH₃), 18.0 (12-CH₃), 18.0 (4-CH₃), 14.3 (2-CH₃), 12.8 (10-CH₃), 11.0 (15-CH₃).

### Example 15.

### 3-Decladinosyl-3-O-(4-nitrophenyl)acyl-9-deoxo-9-dihydro-9a-N-(N'-isopropylcarbamoyl)-9a-aza-9a-homoerythromycin A

To a solution of 4-nitrophenylacetic acid (0.85 g) in dry CH₂Cl₂ (25 ml) TEA (0.65 ml) was added and the reaction mixture was cooled to 2-5 °C. Pyvaloyl chloride (0.57 ml) was added and the reaction mixture was stirred at the same temperature for 30 minutes. To a reaction mixture pyridine (1,27 ml) and the solution of 2'-*O*-Acetyl-3-decladinosyl-9-deoxo-9-dihydro-9a-*N*-(*N*'-isopropylcarbamoyl)-9a-aza-9a-homoerythromycin A from Example 11 (1,00 g) in dry CH₂Cl₂ (5 ml) were added and the reaction mixture was stirred at room temperature for 20 hours. Saturated aqueous solution of NaHCO₃ (30 ml) was added and the layers were separated. The water layer was extracted two more times with CH₂Cl₂. Combined organic extracts were rinsed with brine, dried over K₂CO₃ and evaporated yielding 1,429 g of oily product. The obtained product was dissolved in MeOH (50 ml) and stirred for 24 hours at room temperature.
The solvent was evaporated and the crude product was purified by chromatography on a silica gel column using the system CH₂Cl₂ : MeOH : NH₄OH = 90 : 3 : 0.3. The combining and evaporating of chromatographically homogenous fractions gave the title product (0.45 g) with following physical-chemical constants:
- **TLC**: R_{f}= 0.40 (CH₂Cl₂ : MeOH : NH₄OH = 90 : 9 : 0.5).
- **IR (KBr) [νcm**^{**-1**}**]:**: 3449, 2975, 2939, 2877, 2791, 1741, 1626, 1604, 1523, 1459, 1382,1347,1255,1167,1111,1075, 1051, 1032, 984, 959, 856, 767, 728, 687.
- **MS m/z:**: (ES): MH⁺ = 825
^{**1**}**H NMR (300 MHz, CDCl**_{**3**}**) [δ/ppm]**
8.21 (4"'-H, 6"'-H), 7,54 (3"'-H, 7"'-H), 5.43 (3-H), 5.06 (13-H), 4.42 (9a-NCON'H), 4.23 (1'-H), 3.92 (1 '''-H), 3.60 (5-H), 3.49 (5'-H), 3.26 (2'-H), 2.68 (2-H), 2.45 (3'-H), 2.29 (3'-N(CH₃)₂), 2.20 (8-H), 1.95 (14-Ha), 1.90 (4-H), 1.66 (4'-Ha), 1.50 (14-Hb), 1.32 (6-CH₃), 1.26 (4'-Hb), 1.29 (10-CH₃), 1.22 (5'-CH₃), 1.17 (12-CH₃), 1.05 (4-CH₃), 0.95 (2-CH₃), 1.15 (1"-(CH₃)₂), 1.08 (8-CH₃), 0.87 (15-CH₃).
^{**13**}**C NMR (75 MHz, CDCl**_{**3**}**) [δ/ppm]**
174.7 (1-C), 169.9 (1"-CO), 158.7 (9a-NCONH), 147.1 (5"'-C), 141.5 (2"'-C), 130.5 (3"'-C, 7"'-C), 123.6 (2"'-C, 6"'-C), 103.4 (1'-C), 88.3 (5-C), 78.1 (13-C), 74.5 (3-C), 74.2 (6-C), 70.4 (2'-C), 69.8 (5'-C), 65.4 (3'-C), 44.6 (2-C), 41.3 (1'''-C), 38.4 (4-C), 48.7 (1"-C), 41.3 (7-C), 40.2 (3'-N(CH₃)₂), 28.7 (4'-C), 27.8 (8-C), 27.4 (6-CH₃), 23.2 (1"-(CH₃)₂), 21.7 (14-C), 20.7 (8-CH₃), 20.8 (5'-CH₃), 16.9 (12-CH₃), 15.2 (2-CH₃), 12.5 (10-CH₃), 11.0 (15-CH₃), 8.9 (4-CH₃).

### Example 16.

### 2'-O-Acetyl-3-decladinosyl-9-deoxo-9-dihydro-9a-N-[N'-(2,4-dichlorphenyl)carbamoyl]-9a-aza-9a-homoerythromycin A

To a solution of 3-decladinosyl-9-deoxo-9-dihydro-9a-*N*-[*N*'-(2,4-dichlorphenyl)carbamoyl]-9a-aza-9a-homoerythromycin A (6,60 g) in CH₂Cl₂ (250 ml), NaHCO₃ (3,25 g) and acetic acid anhydride (895µl) were added and it was then stirred for 24 hours at room temperature. In to the reaction mixture a saturated NaHCO₃ solution was added, the layers were separated and the aqueous one was extracted two more times with CH₂Cl₂. The combined organic extracts were rinsed with saturated NaHCO₃ solution and water and evaporated yielding the title product (6,30 g) with following physical-chemical constants:
- **TLC**: R_{f} = 0.33 (CH₂Cl₂ : MeOH : NH₄OH = 90 : 9 : 1,5).
- **IR (KBr) [νcm**^{**-1**}**]:**: 3545, 3448, 3393, 2972, 2940, 2882, 2831, 2787, 1727, 1638, 1586, 1522, 1490, 1460, 1382, 1335, 1309, 1298, 1247, 1201, 1166, 1100, 1057, 1036, 1006, 985, 947, 893, 864, 817, 756, 702, 669, 620.
- **MS m/z:**: (ES): MH⁺ = 806,16

### Example 17.

### 2'-O-Acetyl-3-O-mesyl-3-decladinosyl-9-deoxo-9-dihydro-9a-N-[N'-(2,4-dichlorphenyl)carbamoyl]-9a-aza-9a-homoerythromycin A

To a solution of 2'-*O*-Acetyl-3-decladinosyl-9-deoxo-9-dihydro-9a-*N*-[*N*'-(2,4-dichlorphenyl)carbamoyl]-9a-aza-9a-homoerythromycin A (1,50 g) in pyridine (60 ml) methylsulphonyl anhydride (1,16 g) was added and the reaction mixture was stirred at room temperature for 3 hours. The solvent was evaporated and the residue was dissolved in CH₂Cl₂ (50 ml). Saturated aqueous solution of NaHCO₃ (50 ml) was added, the layers were separated and the water layer was extracted two more times with CH₂Cl₂ Combined organic extracts were rinsed with NaHCO₃ and brine, dried over K₂CO₃ and evaporated yielding 1,62 g of crude product with following physical-chemical constants
- **TLC**: R_{f} = 0,69 (ethylacetate : hexane : diethylamine = 10 : 10 : 2).
- **IR (KBr) [νcm**^{**-1**}**]:**: 3448, 3058, 2935, 1735, 1655, 1637, 1603, 1560, 1528, 1486, 1376, 1332, 1240, 1208, 1193, 1097, 1059, 1001, 916, 822, 785, 773, 753, 681,609.
- **MS m/z:**: (ES): MH⁺ = 884,05

### Example 18.

### 3-Decladinosyl-3,6-hemiketal-9-deoxo-9-dihydro-9a-N-[N'-(2,4-dichlorphenyl)carbamoyl]-9a-aza-9a-homoerythromycin A

To a solution of 2'-*O*-Acetyl-3-decladinosyl-9-deoxo-9-dihydro-9a-*N*-[*N*'-(2,4-dichlorphenyl)carbamoyl]-9a-aza-9a-homoerythromycin A (2,00 g) from Example 16. in CH₂Cl₂ (30 ml) dimethylsulfoxide (2,66 ml) and *N,N*-dimethyl-aminopropyl-ethyl-carbodiimid (2,85 ml) were added. The reaction mixture was cooled to 15 °C, a then, keeping the temperature constant, solution of pyridinium trifluoracetate (2,15 g) in CH₂Cl₂ (10 ml) was added dropwise during 30 minutes. The reaction mixture was stirred at 15 °C to room temperature for additional 2 hours. To the reaction mixture saturated aqueous solution of NaCl (40 ml) was added and the pH value was adjusted to pH 9.5. The layers were separated and the water layer was extracted two more times with CH₂Cl₂ Combined organic extracts were rinsed with brine, NaHCO₃ and water, dried over K₂CO₃ and evaporated yielding 1,10 g of the crude product which is dissolved in MeOH (50 ml) and stirred for 24 hours at room temperature. The solvent was evaporated and the residue purified by low pressure chromatography on a silica gel column using the system CH₂Cl₂ : MeOH : NH₄OH = 90 : 3 : 0,3. The combining and evaporating of chromatographically homogenous fractions gave the title product (0,516 g) with following physical-chemical constants:
- **TLC**: R_{f}= 0.60 (CH₂Cl₂ : MeOH : NH₄OH = 90 : 9 : 0.5).
- **IR (KBr) [vcm**^{**-1**}**]:**: 3448, 2974, 2938, 2877, 2782, 1720, 1667, 1580, 1512, 1460, 1384, 1324, 1299, 1231, 1195, 1115, 1099, 1072, 1049, 962, 862, 823.
- **MS m/z:**: (ES): MH⁺ = 762,12
^{**1**}**H NMR (300 MHz, CDCl**_{**3**}**) [δ/ppm]**
8.14 (6"-H), 7.32 (3"-H), 7.19 (5"-H), 4.91 (13-H), 4.36 (9a-NCON'H), 4.21 (1'-H), 3.80 (5-H), 3.63 (11-H), 3.51 (5'-H), 3.23 (2'-H), 2.59 (10-H), 2.56 (2-H), 2.49 (3'-H), 2.27 (3'-N(CH₃)₂), 2.20 (8-H), 2.09 (4-H), 1.83 (14-Ha), 1.68 (4'-Ha), 1.62 (14-Hb), 1.55 (7-Ha), 1.47 (7-Hb), 1.42 (6-CH₃), 1.30 (2-CH₃), 1.26 (4'-Hb), 1.26 (5'-CH₃), 1.24 (4-CH₃), 1.21 (12-CH₃), 1.19 (10-CH₃), 1.07 (8-CH₃), 0.88 (15-CH₃).
^{**13**}**C NMR (75 MHz, CDCl**_{**3**}**) [δ/ppm]**
177.4 (1-C), 154.8 (9a-NCONH), 134.8 (1"-C), 128.3 (3"-C), 127.3 (5"-C), 127.1 (2"-C), 122.7 (4"-C), 121.9 (6"-C), 106.2 (1'-C), 103.3 (3-C), 94.2 (5-C), 83.6 (6-C), 83.3 (11-C), 79.0 (13-C), 75.6 (12-C), 69.7 (2'-C), 69.6 (5'-C), 65.5 (3'-C), 49.2 (2-C), 48.4 (4-C), 46.3 (10-C), 41.4 (7-C), 40.3 (3'N-(CH₃)₂), 28.6 (4'-C), 28.4 (8-C), 21.6 (14-C), 23.3 (5'-CH₃), 26.4 (6-CH₃), 21.5 (8- CH₃),17.7 (12- CH₃), 16.8 (12-CH₃), 14.2 (10-CH₃), 13.8 (2-CH₃), 12.9 (4-CH₃), 10.9 (15-CH₃).

### Example 19.

### 3-Decladinosyl-3,6-cyclic ether-9-deoxo-9-dihydro-9a-N-[N'-(2,4-dichlorphenyl)carbamoyl]-9a-aza-9a-homoerythromycin A

To a solution of 2'-*O*-Acetyl-3-O-mesyl-3-decladinosyl-9-deoxo-9-dihydro-9a-*N*-[*N*'-(2,4-dichlorphenyl)carbamoyl]-9a-aza-9a-homoerythromycin A from Example 12. (1,50 g) in DMF/THF (57 ml / 19 ml) suspension (60%) of NaH in mineral oil (270 mg) was added and the reaction mixture was stirred at 0 °C for 5 hours, and additional 20h at the room temperature. The reaction mixture was poured into saturated aqueous solution of NaHCO₃ (30 ml), EtOAc (30 ml) was added and the layers were separated. The water layer was extracted two more times with EtOAc. Combined organic extracts were rinsed with NaHCO₃ and brine, dried over K₂CO₃ and evaporated yielding 1,25 g of product. The obtained product was dissolved in MeOH (20 ml) and stirred for 24 hours at room temperature. The solvent was evaporated and the crude product was purified by chromatography on a silica gel column using the system CH₂Cl₂ : MeOH : NH₄OH = 90 : 3 : 0.3. The combining and evaporating of chromatographically homogenous fractions gave the title product (0.27 g) with following physical-chemical constants:
- **TLC**: R_{f}= 0.42 (CH₂Cl₂ : MeOH : NH₄OH = 90 : 9 : 0.5).
- **IR (KBr) [νcm**^{**-1**}**]:**: 3448, 2971, 2936, 2875, 2782, 1736, 1670, 1579, 1510, 1460, 1383, 1327, 1299, 1260, 1177, 1142, 1115, 1099, 1072, 1047, 961, 890, 861, 829, 763, 670, 630.
- **MS m/z:**: (ES): MH⁺ = 746,02
^{**1**}**H NMR (300 MHz, CDCl**_{**3**}**) [δ/ppm]**
8.17 (6"-H), 7.32 (3"-H), 7.18 (5"-H), 4.91 (13-H), 4.23 (9a-NCON'H), 4.20 (1'-H), 3.88 (11-H), 3.56 (5-H), 3.51 (5'-H), 3.18 (2'-H), 2.59 (2-H), 2.52 (3'-H), 2.29 (3'-N(CH₃)₂), 2.18 (8-H), 2.06 (4-H), 1.84 (14-Ha), 1.71 (14-Hb), 1.69 (4'-Ha), 1.63 (7-Ha), 1.44 (4'-Hb), 1.28 (10-CH₃), 1.27 (7-Hb), 1.25 (6-CH₃), 1.23 (4-CH₃), 1.21 (5'-CH₃), 1.20 (2-CH₃), 1.13 (12-CH₃), 1.06 (8-CH₃), 0.89 (15-CH₃).
^{**13**}**C NMR (75 MHz, CDCl**_{**3**}**) [δ/ppm]**
176.1 (1-C), 154.2 (9a-NCONH), 134.4 (1"-C), 127.6 (3"-C), 127.1 (5"-C). 126.4 (2"-C), 122.1 (4"-C), 121.2 (6"-C), 104.3 (1'-C), 93.4 (5-C), 83.3 (11-C), 83.2 (6-C), 82.8 (3-C), 79.1 (13-C), 74.9 (12-C), 69.1 (5'-C), 69.0 (2'-C), 64.9 (3'-C), 45.7 (2-C), 45.2 (4-C), 40.1 (7-C), 39.7 (3'N-(CH₃)₂), 30.0 (4'-C), 29.2 (8-C), 21.2 (14-C), 21.0 (5'-CH₃), 22.7 (6-CH₃), 20.9 (8- CH₃), 12.6 (4-CH₃), 17.0 (12- CH₃), 12.8 (10-CH₃), 13.7 (2-CH₃), 10.4 (15-CH₃).

### Example 20.

### 3-Decladinosyl-3-O-(4-nitrophenyl)acyl-9-deoxo-9-dihydro-9a-N-[N'-(2,4-dichlorphenyl)carbamoyl]-9a-aza-9a-homoerythromycin A

To a solution of 4-nitrophenylacetic acid (0.74 g) in dry CH₂Cl₂ (25 ml) TEA (0.57 ml) was added and the reaction mixture was cooled to 2-5 °C. Pyvaloyl chloride (0.50 ml) was added and the reaction mixture was stirred at the same temperature for 30 minutes. To a reaction mixture pyridine (1,1 ml) and the solution of 2'-*O*-Acetyl-3-decladinosyl-9-deoxo-9-dihydro-9a-*N*-[*N*'-(2,4-dichlorphenyl)carbamoyl]-9a-aza-9a-homoerythromycin A from Example 16 (1,00 g) in dry CH₂Cl₂ (5 ml) were added and the reaction mixture was stirred at room temperature for 20 hours. Saturated aqueous solution of NaHCO₃ (30 ml) was added and the layers were separated. The water layer was extracted two more times with CH₂Cl₂. Combined organic extracts were rinsed with brine, dried over K₂CO₃ and evaporated yielding 1,14 g of oily product. The obtained product was dissolved in MeOH (50 ml) and stirred for 24 hours at room temperature.
The solvent was evaporated and the crude product was purified by chromatography on a silica gel column using the system CH₂Cl₂ : MeOH : NH₄OH = 90 : 3 : 0.3. The combining and evaporating of chromatographically homogenous fractions gave the title product (0.52 g) with following physical-chemical constants:
- **TLC**: R_{f}= 0.55 (CH₂Cl₂ : MeOH : NH₄OH = 90 : 9 : 0.5).
- **IR (KBr) [νcm**^{**-1**}**]:**: 3448, 2975, 2938, 2879, 2789, 1741, 1665, 1607, 1578, 1522, 1459, 1382, 1347, 1298, 1256, 1229, 1165, 1110, 1074, 1050, 983, 958, 857, 821, 731, 685, 669.
- **MS m/z:**: (ES): MH⁺ = 927,2
^{**1**}**H NMR (300 MHz, CDCl**_{**3**}**) [δ/ppm]**
8.18 (4"'-H, 6"'-H), 8.13 (6"-H), 7.51 (3"'-H, 7'''-H), 7,32 (3"-H), 7.18 (5"-H), 5.36 (3-H), 5.03 (13-H), 4.42 (9a-NCON'H), 4.23 (1'-H), 3.80 (1 "'-H), 3.69 (5-H), 3.49 (5'-H), 3.25 (2'-H), 2.68 (2-H), 2.45 (3'-H), 2.32 (3'-N(CH₃)₂), 2.29 (8-H), 1.92 (14-Ha), 1.92 (4-H), 1.66 (4'-Ha), 1.58 (7-Ha), 1.51 (14-Hb), 1.38 (10-CH₃), 1.32 (6-CH₃), 1.26 (7-Hb), 1.18 (4'-Hb), 1.24 (12-CH₃), 1.16 (5'-CH₃), 1.07 (8-CH₃), 0.95 (2-CH₃), 0.94 (4-CH₃), 0.85 (15-CH₃).
^{**13**}**C NMR (75 MHz, CDCl**_{**3**}**) [δ/ppm]**
174.9 (1-C), 169.9 (1"-CO), 154.9 (9a-NCONH), 147.2 (5"'-C), 141,3 (2'''-C), 134.8 (1"-C), 130.6 (3'''-C, 7'''-C), 128.5 (3"-C), 127.4 (2"-C, 5"-C), 123.7 (2'''-C, 6'''-C), 122.6 (4"-C), 122.2 (6"-C), 103.9 (1'-C), 88.3 (5-C), 77.6 (13-C), 74.3 (3-C), 74.2 (6-C), 70.5 (2'-C), 69.8 (5'-C), 65.5 (3'-C), 47.3 (4-C), 44.6 (2-C), 41.2 (1'''-C), 41.2 (7-C), 40.5 (3'-N(CH₃)₂), 29.1 (4'-C), 27.5 (8-C), 27.4 (6-CH₃), 22.0 (14-C), 21.1 (5'-CH₃), 21.0 (8-CH₃), 12.3 (12-CH₃), 13.1 (10-CH₃), 11.2 (15-CH₃), 3.2 (4-CH₃).

### Example 21.

### 3-Decladinosyl-6-O-methyl-9-deoxo-9-dihydro-9a-N-(N-isopropylcarbamoyl)-9a-aza 9a-homoerythromycin A

To a solution of 3-decladinosyl-6-*O*-methyl-9-deoxo-9-dihydro-9a-aza-9a-homoerythromycin A (0,20 g) in acetonitrile (20 ml) isopropylisocyanate (0,07 ml) was added. Reaction mixture was stirred for 2 hours at room temperature. The solvent was evaporated and 0,25 g of crude product is obtained. Crude product was purified by chromatography on a silica gel column using the system (CH₂Cl₂ : MeOH : NH₄OH = 90 : 5 : 0.5). The combining and evaporating of chromatographically homogenous fractions gave the title product (0.128 g) with following physical-chemical constants:
- **TLC**: R_{f}= 0.54 (CHCl₃ : MeOH : NH₄OH = 6 : 1 : 0.1).
- **IR (KBr) [νcm**^{**-1**}**]:**: 3424, 2975, 2937, 2877, 1732, 1687, 1627, 1562, 1525, 1460, 1379,1270,1166,1112,1080,1053,984,958,938, 896, 828, 766.
- **MS m/z:**: **(ES): MH**^{**+**} = 676,8
^{**1**}**H NMR (300 MHz, CDCl**_{**3**}**) [δ/ppm]**
5.16 (13-H), 4.44 (1'-H), 3.91 (1"-H), 3.65 (3-H), 3.73 (5-H), 3.53 (5'-H), 3.23 (2'-H), 3.14 (6-OCH₃), 2.61 (2-H), 2.48 (3'-H), 2.25 (3'-N(CH₃)_{2,}), 1.82 (4-H), 1.91 (14a-H), 2.03 (8-H), 1.66 (4'a-H), 1.49 (14b-H), 1.35 (18-CH₃), 1.28 (16-CH₃), 1.25 (20-CH₃), 1.25 (5'-CH₃), 1.25 (4'b-H), 1.15 (1"-(CH₃)₂), 1.14 (21-CH₃), 1.05 (17-CH₃), 0.96 (19-CH₃), 0.88 (15-CH₃).
^{**13**}**C NMR (75 MHz, CDCl**_{**3**}**) [δ/ppm]**
175.8 (1-C), 155.1 (9a-NCONH), 106.6 (1'-C), 90.2 (5-C), 79.5 (6-C), 78.9 (3-C), 74.2 (11-C), 74.7 (12-C), 70.5 (2'-C), 70.0 (5'-C), 65.7 (3'-C), 49.8 (6-OCH₃), 44.8 (2-C), 42.7 (1"-C), 40.3 (3'N-(CH₃)₂), 36.4 (4-C), 28.1 (4'-C), 27.5 (8-C), 23.6 (18-CH₃), 23.3 (1"-(CH₃)₂), 22.3 (14-C), 21.3 (5'-CH₃), 20.6 (19-CH₃), 16.9 (21-CH₃), 15.5 (16-CH₃), 12.6 (20-CH₃), 11.2 (15-CH₃), 7.7 (17-CH₃).

### Example 22.

### 2'-O-Acetyl-3-decladinosyl-6-O-methyl-9-deoxo-9-dihydro-9a-N-(N-isopropylcarbamoyl)-9a-aza-9a-homoerythromycin A

To a solution of 3-decladinosyl-6-*O*-methyl-9-deoxo-9-dihydro-9a-*N*-(*N-*isopropylcarbamoyl)-9a-aza-9a-homoerythromycin A (1,08 g) in CH₂Cl₂ (20 ml), NaHCO₃ (0,54 g) and acetic acid anhydride (166µl) were added and it was then stirred for 20 hours at room temperature. Onto the reaction mixture a saturated NaHCO₃ solution was added, the layers were separated and the aqueous one was extracted two more times with CH₂Cl₂. The combined organic extracts were rinsed with saturated NaHCO₃ solution and water and evaporated yielding the title product (0,68 g) with following physical-chemical constants:
- **TLC**: R_{f}= 0.57 (CHCl₃ : MeOH : NH₄OH = 6 : 1 : 0.1).
- **IR (KBr) [vcm**^{**-1**}**]:**: 3431, 2974, 2937, 2876, 1734, 1629, 1524, 1459, 1376, 1243, 1167, 1083, 1058, 985, 957, 938, 904, 806, 671.
- **MS m/z:**: **(ES): MH**^{**+**} = 718,22

### Example 23.

### 2'-O-Acetyl-3-O-mesyl-6-O-methyl-3-decladinosyl-9-deoxo-9-dihydro-9a-N-(N-isopropylcarbamoyl)-9a-aza-9a-homoerythromycin A

To a solution of 2'-*O*-Acetyl-3-decladinosyl-6-*O*-methyl-9-deoxo-9-dihydro-9a-*N*-(*N-*isopropylcarbamoyl)-9a-aza-9a-homoerythromycin A (0,35 g) in pyridine (15 ml) methylsulphonyl anhydride (0,30 g) was added and the reaction mixture was stirred at room temperature for 3 hours. The solvent was evaporated and the residue was dissolved in CH₂Cl₂ (20 ml). Saturated aqueous solution of NaHCO₃ (20 ml) was added, the layers were separated and the water layer was extracted two more times with CH₂Cl₂ Combined organic extracts were rinsed with NaHCO₃ and brine, dried over K₂CO₃ and evaporated yielding 0,36 g of crude product with following physical-chemical constants
- **TLC**: R_{f}= 0.76 (CHCl₃ : MeOH : NH₄OH = 6 : 1 : 0.1).
- **IR (KBr) [νcm**^{**-1**}**]:**: 3427, 2975, 2932, 2877,2854, 1739, 1628, 1524, 1462, 1375, 1342,1243,1175, 1112, 1060, 958, 917, 830, 768, 707, 669.
- **MS m/z:**: **(ES): MH**^{**+**} = 796,28

### Example 24.

### 3-keto-3-decladinosyl-6-O-methyl-9-deoxo-9-dihydro-9a-N-(N⁻-isopropylcarbamoyal)-9a-aza-9a-homoerythromycin A

To a solution of 2'-*O*-Acetyl-3-decladinosyl-6-*O*-methyl-9-deoxo-9-dihydro-9a-*N*-(*N-*isopropylcarbamoyl)-9a-aza-9a-homoerythromycin A (0,18 g) in CH₂Cl₂ (10 ml) dimethylsulfoxide (0,21 ml) and *N,N*-dimethyl-aminopropyl-ethyl-carbodiimid (0,28 ml) were added. The reaction mixture was cooled to 15 °C, and then, keeping the temperature constant, solution of pyridinium trifluoracetate (0,22 g) in CH₂Cl₂ (5 ml) was added dropwise during 30 minutes. The reaction mixture was stirred at 15 °C to room temperature for additional 2 hours. To the reaction mixture saturated aqueous solution of NaCl (20 ml) was added and the pH value was adjusted to pH 9.5. The layers were separated and the water layer was extracted two more times with CH₂Cl₂ Combined organic extracts were rinsed with brine, NaHCO₃ and water, dried over K₂CO₃ and evaporated yielding 0,15 g of the crude product which is dissolved in MeOH (20 ml) and stirred for 24 hours at room temperature. The solvent was evaporated and the residue purified by low pressure chromatography on a silica gel column using the system CH₂Cl₂ : MeOH : NH₄OH = 90 : 3 : 0,3. The combining and evaporating of chromatographically homogenous fractions gave the title product (0,074 g) with following physical-chemical constants:
- **TLC**: R_{f}= 0.32 (CH₂Cl₂ : MeOH : NH₄OH = 90 : 9 : 0.5).
- **IR (KBr) [νcm**^{**-1**}**]:**: 3428, 2936, 1741, 1629, 1520, 1458, 1378, 1260, 1172, 1111, 1077,1050,985,810.
- **MS m/z:**: **(ES): MH**^{**+**} = 674,27
^{**1**}**H NMR (300 MHz, CDCl**_{**3**}**) [δ/ppm]**
5.11 (13-H), 4.42 (1'-H), 4.34 (5-H). 3.91 (1"-H), 3.82 (2-H), 3.63 (5'-H), 3.21 (2'-H), 3.12 (4-H), 2.99 (6-OCH₃), 2.57 (3'-H), 2.33 (3'-N(CH₃)_{2,}), 2.04 (8-H), 1.94 (14a-H), 1.75 (4'a-H), 1.54 (14b-H), 1.38 (18-CH₃), 1.33 (16-CH₃), 1.26 (20-CH₃), 1.27 (5'-CH₃), 1.27 (4'b-H), 1.16 (1"-(CH₃)₂), 1.14 (21-CH₃), 1.27 (17-CH₃), 0.98 (19-CH₃), 0.90 (15-CH₃).
^{**13**}**C NMR (75 MHz, CDCl**_{**3**}**) [δ/ppm]**
207.0 (3-C), 170.6 (1-C), 158.0 (9a-NCONH), 102.8 (1'-C), 75.8 (5-C), 79.4 (6-C), 74.4 (12-C), 74.2 (11-C), 70.3 (2'-C), 69.2 (5'-C), 65.8 (3'-C), 50.7 (2-C), 50.1 (6-OCH₃), 45.7 (4-C), 42.8 (1"-C), 40.4 (3'N-(CH₃)₂), 29.1 (4'-C), 27.4 (8-C), 23.8 (18-CH₃), 23.2 (1"-(CH₃)₂), 22.4 (14-C), 21.2 (5'-CH₃), 20.8 (19-CH₃), 16.8 (21-CH₃), 14.0 (16-CH₃), 13.2 (20-CH₃), 12.5 (17-CH₃), 11.3 (15-CH₃).

### Example 25.

### 2,3-anhydro-3-decladinosyl-6-O-methyl-9-deoxo-9-dihydro-9a-N-(N-isopropylcarbamoyl)-9a-aza-9a-homoerythromycin A

To a solution of 2'-*O*-Acetyl-3-O-mesyl-3-decladinosyl-6-*O*-methyl-9-deoxo-9-dihydro-9a*N*-(*N*'-isopropylcarbamoyl)-9a-aza-9a-homoerythromycin A (0,35 g) in DMF/THF (17 ml/5 ml) suspension (60%) of NaH in mineral oil (90 mg) was added and the reaction mixture was stirred at 0 °C for 5 hours. The reaction mixture was poured into saturated aqueos solution of NaHCO₃ (20 ml), EtOAc (20 ml) was added and the layers were separated. The water layer was extracted two more times with EtOAc. Combined organic extracts were rinsed with NaHCO₃ and brine, dried over K₂CO₃ and evaporated yielding 0.53 g of product. The obtained product was dissolved in MeOH (20 ml) and stirred for 24 hours at room temperature. The solvent was evaporated and the crude product was purified by chromatography on a silica gel column using the system (CH₂Cl₂ : MeOH : NH₄OH = 90 : 3 : 0.3). The combining and evaporating of chromatographically homogenous fractions gave the title product (0.031 g) with following physical-chemical constants:
- **TLC**: R_{f}= 0.50 (CHCl₃: MeOH : NH₄OH = 6 : 1 : 0.1).
- **IR (KBr) [νcm**^{**-1**}**]:**: 3439, 2974, 2937, 1735, 1628, 1520, 1459, 1381, 1336, 1270, 1175,1111,1075,1051,958,919,832,766,535.
- **MS m/z:**: **(ES): MH**^{**+**} = 658,22
^{**1**}**H NMR (300 MHz, CDCl**_{**3**}**) [δ/ppm]**
5.16 (13-H), 4.93 (3-H), 4.56 (1'-H), 3.90 (1"-H), 3.23 (5-H), 3.53 (5'-H), 3.27 (2'-H), 3.12 (6-OCH₃), 2.70 (3'-H), 2.36 (3'-N(CH₃)₂), 1.52 (4-H), 1.91 (14a-H), 2.05 (8-H), 1.70 (4'a-H), 1.49 (14b-H), 1.36 (18-CH₃), 1.30 (16-CH₃), 1.25 (20-CH₃), 1.25 (5'-CH₃), 1.25 (4'b-H), 1.15 (1"-(CH₃)₂), 1.12 (21-CH₃), 1.06 (17-CH₃), 0.96 (19-CH₃), 0.88 (15-CH₃).
^{**13**}**C NMR (75 MHz, CDCl**_{**3**}**) [δ/ppm]**
173.2 (1-C), 158.1 (9a-NCONH), 146.4 (2-C), 127.4 (3-C), 106.7 (1'-C), 86.1 (5-C), 79.5 (6-C), 74.2 (11-C), 75.0 (12-C), 70.7 (2'-C), 68.3 (5'-C), 65.9 (3'-C), 50.7 (6-OCH₃), 42.8 (1"-C), 39.0 (3'N-(CH₃)₂), 40.4 (4-C), 29.1 (4'-C), 28.4 (8-C), 23.4 (18-CH₃), 22.3 (14-C), 22.1 (1"-(CH₃)₂), 20.9 (5'-CH₃), 20.5 (19-CH₃), 16.8 (21-CH₃), 14.2 (16-CH₃), 12.5 (20-CH₃), 11.4 (15-CH₃), 8.8 (17-CH₃).

### Example 26.

### 3-Dedadinosyl-6-O-methyl-9-deoxo-9-dihydro-9a-N-[N⁻-(2,4-dichlorphenyl)carbamoyl] -9a-aza-9a-homoerythromycin A

To a solution of 3-decladinosyl-6-*O*-methyl-9-deoxo-9-dihydro-9a-aza-9a-homoerythromycin A (0,20 g) in acetonitrile (20 ml) 2,4-dichlorphenylisocyanate (0,064 6) was added. Reaction mixture was stirred for 30 min at room temperature. The solvent was evaporated and 0,254 g of crude product is obtained. Crude product was purified by chromatography on a silica gel column using the system (CH₂Cl₂ : MeOH : NH₄OH = 90 : 5 : 0.5). The combining and evaporating of chromatographically homogenous fractions gave the title product (0.169 g) with following physical-chemical constants:
- **TLC**: R_{f}= 0.61 (CHCl₃: MeOH : NH₄OH = 6: 1 : 0.1).
- **IR (KBr) [vcm**^{**-1**}**]:**: 3448, 2975, 2939, 2879, 2787, 1729, 1707, 1670, 1582, 1517, 1459, 1382, 1328, 1298, 1274, 1165, 1112, 1076, 1051, 983, 956, 937, 897, 861, 822, 750, 699.
- **MS m/z:**: **(ES): MH**^{**+**} = 778,6
^{**1**}**H NMR (300 MHz, CDCl**_{**3**}**) [δ/ppm]** 8.13 (6"-H), 7.34 (3"-H), 7.18 (5"-H), 5.11 (13-H), 4.39 (1'-H), 3.81 (5-H), 3.72 (3-H), 3.52 (5'-H), 3.35 (6-OCH₃), 3.18 (2'-H), 2.61 (2-H), 2.61 (10-H), 2.50 (3'-H), 2.27 (3'-N(CH₃)₂), 2.04 (8-H), 1.93 (14a-H), 1.89 (4-H), 1.68 (4'a-H), 1.52 (14b-H), 1.35 (6-CH₃), 1.33 (4'b-H), 1.28 (2-CH₃), 1.25 (10-CH₃), 1.23 (5'- CH₃), 1.18 (12-CH₃), 1.04 (4-CH₃), 1.02 (8-CH₃), 0.89 (15-CH₃).
^{**13**}**C NMR (75 MHz, CDCl**_{**3**}**) [δ/ppm]**
176.3 (1-C), 155.0 (9a-NCONH), 134.9 (1"-C), 128.4 (3"-C), 127.7 (5"-C), 127.1 (2"-C), 122.7 (4"-C), 122.1 (6"-C), 106.9 (1'-C), 90.3 (5-C), 79.7 (3-C), 79.1 (6-C), 76.1 (13-C), 73.6 (11-C), 73.6 (12-C), 70.5 (2'-C), 69.9 (5'-C), 65.7 (3'-C), 49.9 (6-O-CH₃), 44.7 (2-C), 40.3 (3'N-(CH₃)₂), 36.7 (4-C), 35.5 (7-C), 28.3 (4'-H), 28.2 (8-C), 22.4 (14-C), 21.3 (5'-CH₃), 20.6 (18-CH₃), 16.9 (21-CH₃), 15.4 (16-CH₃), 20.5 (20-CH₃), 11.4 (15-CH₃), 7.6 (17-CH₃).

### Example 27.

### 2'-O-Acetyl-3-decladinosyl-6-O-methyl-9-deoxo-9-dihydro-9a-N-[N⁻-(2,4-dichlorphenyl)carbamoyl]-9a-aza-9a-homoerythromycin A

To a solution of 3-decladinosyl-6-*O*-methyl-9-deoxo-9-dihydro-9a-*N*-[*N*⁻-(2,4-dichlorphenyl)carbamoyl]-9a-aza-9a-homoerythromycin A (0,12 g) in CH₂Cl₂ (10 ml), NaHCO₃ (0,058 g) and acetic acid anhydride (15µl) were added and it was then stirred for 24 hours at room temperature. Onto the reaction mixture a saturated NaHCO₃ solution was added, the layers were separated and the aqueous one was extracted two more times with CH₂Cl₂. The combined organic extracts were rinsed with saturated NaHCO₃ solution and water and evaporated yielding the title product (0,115 g) with following physical-chemical constants:
- **TLC**: R_{f}= 0.29 (CHCl₃ : MeOH : NH₄OH = 90 : 9 : 0.5).
- **IR (KBr) [vcm**^{**-1**}**]:**: 3449, 2973, 2939, 1748, 1730, 1668, 1582, 1517, 1459, 1377, 1298, 1261, 1239, 1165, 1098, 1050, 985, 906, 866, 809, 764, 664, 622, 584, 544, 505, 483, 463, 444, 386.
- **MS m/z:**: (ES): MH⁺ = 820,19

### Example 28.

### 2'-O-Acetyl-3-O-mesyl-6-O-methyl-3-decladinosyl-9-deoxo-9-dihydro-9a-N-[N⁻-(2,4-dichlorphenyl)carbamoyl]-9a-aza-9a-homoerythromycin A

To a solution of 2'-*O*-Acetyl-3-decladinosyl-6-*O*-methyl-9-deoxo-9-dihydro-9a-*N*-[*N*⁻-*(2,4-*dichlorphenyl)carbamoyl]-9a-aza-9a-homoerythromycin A (1,50 g) in pyridine (60 ml) methylsulphonyl anhydride (1,16 g) was added and the reaction mixture was stirred at room temperature for 3 hours. The solvent was evaporated and the residue was dissolved in CH₂Cl₂ (50 ml). Saturated aqueous solution of NaHCO₃ (50 ml) was added, the layers were separated and the water layer was extracted two more times with CH₂Cl₂ Combined organic extracts were rinsed with NaHCO₃ and brine, dried over K₂CO₃ and evaporated yielding 1,57 g of crude product with following physical-chemical constants
- **TLC**: R_{f}= 0.68 (ethylacetate : hexane : diethylamine = 10 : 10 : 2).
- **IR (KBr) [νcm**^{**-1**}**]:**: 3434, 3060, 2974, 2935, 1736, 1701, 1686, 1655, 1637, 1560, 1528, 1509, 1486, 1376, 1330, 1240, 1208, 1193, 1059, 785, 773, 753, 681, 609.
- **MS m/z:**: **(ES): MH**^{**+**} = 898,09

### Example 29.

### 3-keto-3-decladinosyl-6-O-methyl-9-deoxo-9-dihydro-9a-N-[N⁻-(2,4-dichlorphenyl)carbamoyl]-9a-aza-9a-homoerythromycin A

To a solution of 2'-*O*-Acetyl-3-decladinosyl-6-*O*-methyl-9-deoxo-9-dihydro-9a-*N*-[*N*^{*-*}-*(2,4-*dichlorphenyl)carbamoyl]-9a-aza-9a-homoerythromycin A (0,73 g) in CH₂Cl₂ (20 ml) dimethylsulfoxide (1,00 ml) and *N,N*-dimethyl-aminopropyl-ethyl-carbodiimid (1,02 6) were added. The reaction mixture was cooled to 15 °C, and then, keeping the temperature constant, solution of pyridinium trifluoracetate (1,03 g) in CH₂Cl₂ (20 ml) was added drop wise during 30 minutes. The reaction mixture was stirred at 15 °C to room temperature for additional 10 hours. To the reaction mixture saturated aqueous solution of NaCl (20 ml) was added and the pH value was adjusted to pH 9.5. The layers were separated and the water layer was extracted two more times with CH₂Cl₂. Combined organic extracts were rinsed with brine, NaHCO₃ and water, dried over K₂CO₃ and evaporated yielding 0,63 g of the crude product which is dissolved in MeOH (20 ml) and stirred for 24 hours at room temperature. The solvent was evaporated and the residue purified by low pressure chromatography on a silica gel column using the system (CH₂Cl₂ : MeOH : NH₄OH = 90 : 5 : 0,5. The combining and evaporating of chromatographically homogenous fractions gave the title product (0,253 g) with following physical-chemical constants:
- **TLC**: R_{f}= 0.61 (CHCl₃ : MeOH : NH₄OH = 6 : 1 : 0.1).
- **IR (KBr) [νcm**^{**-1**}**]:**: 3448, 2976, 2938, 2877, 2786, 1741, 1719, 1665, 1580, 1515, 1459, 1381, 1298, 1230, 1197, 1167, 1147, 1110, 1076, 1051, 986, 957, 937, 895, 859, 821, 761, 753, 622.
- **MS m/z:**: **(ES): MH**^{**+**} = 776,6
^{**1**}**H NMR (300 MHz; CDCl**_{**3**}**) [δ/ppm]**
8.10 (6"-H), 7.34 (3"-H), 7.18 (5"-H), 5.08 (13-H), 4.40 (1'-H), 4.31 (5-H), 3.81 (2-H), 3.62 (5'-H), 3.44 (6-OCH₃), 3.17 (4-H), 3.16 (2'-H), 2.75 (10-H), 2.49 (3'-H), 2.28 (3'-N(CH₃)₂), 2.19 (8-H), 1.94 (14a-H), 1.68 (4'a-H), 1.56 (14b-H), 1.32 (6-CH₃), 1.30 (2-CH₃), 1.27 (7a-H), 1.24 (10-CH₃), 1.23 (4'b-H), 1.23 (5'- CH₃), 1.19 (12-CH₃), 1.06 (4-CH₃), 1.03 (7b-H), 1.02 (8-CH₃), 0.91 (15-CH₃).
^{**13**}**C NMR (75 MHz, CDCl**_{**3**}**) [δ/ppm]**
207.1 (3-C), 171.1 (1-C), 155.6 (9a-NCONH), 135.1 (1"-C), 128.8 (3"-C), 128.2 (5"-C), 127.8 (2"-C), 123.1 (4"-C), 122.5 (6"-C), 103.1 (1'-C), 79.9 (6-C), 79.5 (13-C), 76.2 (5-C), 73.9 (12-C), 73.4 (11-C), 70.8 (2'-C), 69.7 (5'-C), 65.9 (3'-C), 51.1 (2-C), 50.9 (4-C), 50.6 (6-0-CH₃), 40.8 (3'N-(CH₃)₂), 39.0 (7-C), 29.3 (4'-H), 28.6 (8-C), 22.7 (14-C), 21.6 (5'-CH₃), 21.3 (19-CH₃), 17.2 (21-CH₃), 13.6 (16-CH₃), 12.8 (20-CH₃), 11.6 (15-CH₃), 7.6 (17-CH₃).

### Example 30.

### 2,3-anhydro-3-decladinosyl-6-O-methyl-9-deoxo-9-dihydro-9a-N-[N⁻-(2,4-dichlorphenyl)carbamoyl]-9a-aza-9a-homoerythromycin A

To a solution of 2'-*O*-Acetyl-3-O-mesyl-3-decladinosyl-6-*O*-methyl-9-deoxo-9-dihydro-9a*N*-[*N*'-(2,4-dichlorphenyl)carbamoyl]-9a-aza-9a-homoerythromycin A (1,00 g) in DMF/THF (45 ml/13 ml) suspension (60%) of NaH in mineral oil (400 mg) was added and the reaction mixture was stirred at 0 °C. The reaction mixture was poured into saturated aqueos solution of NaHCO₃ (50 ml), EtOAc (50 ml) was added and the layers were separated. The water layer was extracted two more times with EtOAc. Combined organic extracts were rinsed with NaHCO₃ and brine, dried over K₂CO₃ and evaporated yielding 0.32 g of product. The obtained product was dissolved in MeOH (20 ml) and stirred for 24 hours at room temperature. The solvent was evaporated and the crude product was purified by chromatography on a silica gel column using the system (CH₂Cl₂ : MeOH : NH₄OH = 90 : 3 : 0.3). The combining and evaporating of chromatographically homogenous fractions gave the title product (0.024 g) with following physical-chemical constants:
- **TLC**: R_{f}= 0.43 (ethylacetate : hexane : diethylamine = 10 : 10 : 2).
- **IR (KBr) [νcm**^{**-1**}**]:**: 3452, 2981, 2941, 1748, 1731, 1662, 1582, 1516, 1459, 137, 1298, 1261, 1243, 1165, 1098, 1052, 985, 906, 867, 810, 764, 666, 625, 587.
- **MS m/z:**: **(ES): MH**^{**+**} **=** 760,70
^{**1**}**H NMR (300 MHz, CDCl**_{**3**}**) [δ/ppm]**
8.12 (6"-H), 7.34 (3"-H), 7.18 (5"-H), 5.16 (13-H), 4.92 (3-H), 4.39 (1'-H), 3.20 (5-H), 3:52 (5'-H), 3.30 (6-OCH₃), 3.18 (2'-H), 2.61 (10-H), 2.50 (3'-H), 2.27 (3'-N(CH₃)₂), 2.04 (8-H), 1.93 (14a-H), 1.68 (4'a-H), 1.55 (4-H), 1.52 (14b-H), 1.35 (6-CH₃), 1.29 (2-CH₃), 1.26 (4'b-H), 1.25 (10-CH₃), 1.25 (5'- CH₃), 1.13 (12-CH₃), 1.04 (4-CH₃), 0.97 (8-CH₃), 0.89 (15-CH₃).
^{**13**}**C NMR(75 MHz, CDCl**_{**3**}**) [δ/ppm]**
176.3 (1-C), 156.0 (9a-NCONH), 146.8 (2-C), 127.7 (3-C), 134.8 (1"-C), 128.3 (3"-C), 127.5 (5"-C), 127.0 (2"-C), 122.8 (4"-C), 122.1 (6"-C), 106.9 (1'-C), 86.3 (5-C), 79.1 (6-C), 76.1 (13-C), 73.4 (11-C), 73.5 (12-C), 70.7 (2'-C), 69.9 (5'-C), 65.7 (3'-C), 50.0 (6-*O*-CH₃), 40.3 (3'N-(CH₃)₂), 40.7 (4-C), 35.4 (7-C), 28.3 (4'-H), 28.2 (8-C), 22.4 (14-C), 21.3 (5'-CH₃), 20.6 (8-CH₃), 16.9 (21-CH₃), 14.2 (2-CH₃), 12.8 (20-CH₃), 11v4 (15-CH₃), 7.6 (4-CH₃).

## Claims

1. Novel 3-decladinosyl derivatives of 9a-*N*-carbamoyl- and 9a-*N*-thiocarbamoyl-9-deoxo-9-dihydro-9a-aza-9a-homoerythromycin A of the general formula (I), 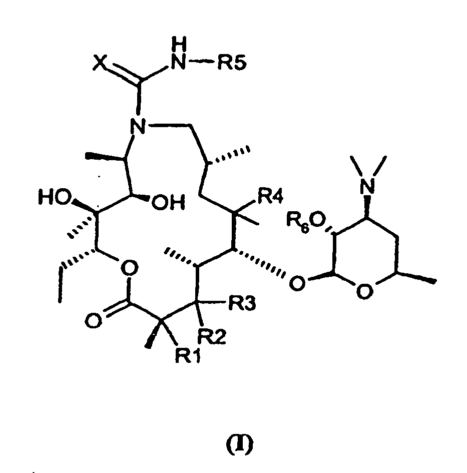 their pharmaceutically acceptable addition salts with inorganic or organic acids and their hydrates,
wherein
R₁ individually stands for hydrogen or together with R₂ stands for double bond,
R₂ individually stands for hydrogen, hydroxyl or a group of the formula **(II),** 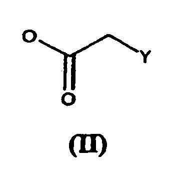 wherein
Y individually stands for monocyclic aromatic ring, unsubstituted or substituted with groups which are selected independently from halogen, OCH₃, NO₂, NH₂
or
R₂ together with R₃ stands for ketone or together with R₁ stands for double bond,
R₃ individually stands for hydrogen or together with R₂ stands for ketone or together with R₄ stands for ether,
R₄ individually stands for hydroxyl, OCH₃ group or together with R₃ stands for ether,
R₅ individually stands for C₁-C₄ alkyl group, C₂-C₄ alkenyl group, -(CH₂)ₘ-Ar, wherein Ar individually stands for phenyl or phenyl substituted with one or two groups which are selected independently from halogen or halogen alkyl, and m is 0-3,
R₆ individually stands for hydrogen or hydroxyl protecting group,
X stands for oxygen or sulphur

2. Compound according to claim 1. **characterized in that** R₁ and R₃ stand for hydrogen, R₂ stands for hydroxyl, R₄ individually stands for hydroxyl or OCH₃ group, R₅ individually stands for C₁-C₄ alkyl group, C₂-C₄ alkenyl group, -(CH₂)ₘ-Ar, wherein Ar individually stands for phenyl or phenyl substituted with one or two groups which are selected independently from halogen or halogen alkyl, m is 0-3, X stands for oxygen or sulphur and R₆ stands for hydrogen.

3. Compound according to claim 2, **characterized in that** R₄ stands for hydroxyl, R₅ stands for ethyl group and X stands for oxygen.

4. Compound according to claim 2, **characterized in that** R₄ stands for hydroxyl, R₅ stands for t-butyl group and X stands for oxygen.

5. Compound according to claim 2, **characterized in that** R₄ stands for hydroxyl, R₅ stands for isopropyl group and X stands for oxygen.

6. Compound according to claim 2, **characterized in that** R₄ stands for OCH₃ group, R₅ stands for isopropyl group and X stands for oxygen.

7. Compound according to claim 2, **characterized in that** R₄ stands for hydroxyl, R₅ stands for allyl group and X stands for sulphur.

8. Compound according to claim 2, **characterized in that** R₄ stands for hydroxyl, R₅ stands for benzyl group and X stands for oxygen.

9. Compound according to claim 2, **characterized in that** R₄ stands for hydroxyl, R₅ stands for benzyl group and X stands for sulphur.

10. Compound according to claim 2, **characterized in that** R₄ stands for hydroxyl, R₅ stands for 2-trifluormethylphenyl group and X stands for oxygen.

11. Compound according to claim 2, **characterized in that** R₄ stands for hydroxyl, R₅ stands for 3-trifluormethylphenyl group and X stands for oxygen.

12. Compound according to claim 2, **characterized in that** R₄ stands for hydroxyl, R₅ stands for 3-trifluormethylphenyl group and X stands for sulphur:

13. Compound according to claim 2, **characterized in that** R₄ stands for OCH₃ group, R₅ stands for 2,4-dichlorphenyl group and X stands for oxygen.

14. Compound according to claim 2, **characterized in that** R₄ stands for hydroxyl, R₅ stands for 2,4-dichlorphenyl group and X stands for oxygen.

15. Compound according to claim 1. **characterized in that** R₁ and R₃ stand for hydrogen, R₂ stands for a group of the formula (II), wherein Y individually stands for monocyclic aromatic ring, unsubstituted or substituted with groups which are selected independently from halogen, OH, OCH₃, NO₂, NH₂, R₄ individually stands for hydroxyl or for OCH₃ group, R₅ individually stands for C₁-C₄ alkyl group, C₂-C₄ alkenyl group, -(CH₂)ₘ-Ar, wherein Ar individually stands for phenyl or phenyl substituted with one or two groups which are selected independently from halogen or halogen alkyl, m is 0-3, X stands for oxygen or sulphur and R₆ stands for hydrogen.

16. Compound according to claim 15, **characterized in that** Y stands for 4-NO₂ substituted phenyl, R₄ stands for hydroxyl, R₅ stands for isopropyl group and X stands for oxygen.

17. Compound according to claim 15, **characterized in that** Y stands for 4-NO₂ substituted phenyl, R₄ stands for hydroxyl, R₅ stands for 2,4-dichlorphenyl group and X stands for oxygen.

18. Compound according to claim 1, **characterized in that** R_{1,} R₂ and R₆ stand for hydrogen, R₃ together with R₄ stands for ether, R₅ individually stands for C₁-C₄ alkyl group, C₂-C₄ alkenyl group, -(CH₂)ₘ-Ar, wherein Ar individually stands for phenyl or phenyl substituted with one or two groups which are selected independently from halogen or halogen alkyl, m is 0-3, X stands for oxygen or sulphur.

19. Compound according to claim 18, **characterized in that** R₅ stands for isopropyl group and X stands for oxygen.

20. Compound according to claim 18, **characterized in that** R₅ stands for 2,4-dichlorphenyl group and X stands for oxygen.

21. Compound according to claim 1, **characterized in that** R₁ and R₆ stand for hydrogen, R₂ stands for hydroxyl, R₃ together with R₄ stands for ether, R₅ individually stands for C₁-C₄ alkyl group, C₂-C₄ alkenyl group, -(CH₂)ₘ-Ar, wherein Ar individually stands for phenyl or phenyl substituted with one or two groups which are selected independently from halogen or halogen alkyl, m is 0-3 and X stands for oxygen or sulphur.

22. Compound according to claim 21, **characterized in that** R₅ stands for isopropyl group and X stands for oxygen.

23. Compound according to claim 21, **characterized in that** R₅ stands for 2,4-dichlorphenyl group and X stands for oxygen.

24. Compound according to claim 1, **characterized in that** R₁ and R₆ stand for hydrogen, R₂ together with R₃ stands for ketone, R₄ stands for OCH₃ group, R₅ individually stands for C₁-C₄ alkyl group, C₂-C₄ alkenyl group, -(CH₂)ₘ-Ar, wherein Ar individually stands for phenyl or phenyl substituted with one or two groups which are selected independently from halogen or halogen alkyl, m is 0-3 and X stands for oxygen or sulphur.

25. Compound according to claim 24, **characterized in that** R₅ stands for isopropyl group and X stands for oxygen.

26. Compound according to claim 24, **characterized in that** R₅ stands for 2,4-dichlorphenyl group and X stands for oxygen.

27. Compound according to claim 1, **characterized in that** R₁ together with R₂ stands for double bond, R₃ and R₆ stand for hydrogen, R₄ stands for OCH₃ group, R₅ individually stands for C₁-C₄ alkyl group, C₂-C₄ alkenyl group, -(CH₂)ₘ-Ar, wherein Ar individually stands for phenyl or phenyl substituted with one or two groups which are selected independently from halogen or halogen alkyl, m is 0-3 and X stands for oxygen or sulphur.

28. Compound according to claim 27, **characterized in that** R₅ stands for isopropyl group and X stands for oxygen.

29. Compound according to claim 24, **characterized in that** R₅ stands for 2,4-dichlorphenyl group and X stands for oxygen.

30. A process for preparation of compounds of the formula (I), 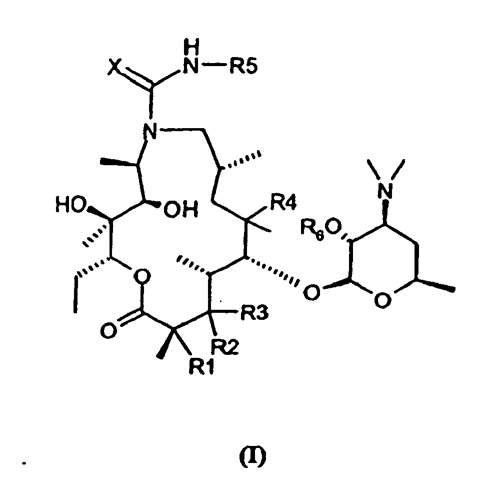 their pharmaceutically acceptable addition salts with inorganic or organic acids and their hydrates,
wherein
R₁ individually stands for hydrogen or together with R₂ stands for double bond,
R₂ individually stands for hydrogen, hydroxyl or a group of the formula **(II),** 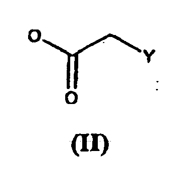 wherein
Y individually stands for monocyclic aromatic ring, unsubstituted or substituted with groups which are selected independently from halogen, OH, OCH₃, NO₂, NH₂
or
R₂ together with R₃ stands for ketone or together with R₁ stands for double bond,
R₃ individually stands for hydrogen or together with R₂ stands for ketone or together with R₄ stands for ether,
R₄ individually stands for hydroxyl, OCH₃ group or together with R₃ stands for ether,
R₅ individually stands for C₁-C₄ alkyl group, C₂-C₄ alkenyl group, -(CH₂)ₘ-Ar, wherein Ar individually stands for phenyl or phenyl substituted with one or two groups which are selected independently from halogen or halogen alkyl and m is 0-3,
R₆ individually stands for hydrogen or hydroxyl protecting group,
X stands for oxygen or sulphur, **characterised in that**
a) starting compounds of the formula 1 (scheme 1.) wherein R₄ individually stands for hydroxyl or OCH₃ group are subjected to a reaction with isocyanate or isothiocyanate of the formula R₅-N=C=X wherein R₅ and X have the above meanings, in a reaction-inert solvent preferably toluene or acetonitril, at a temperature from room temperature to the reflux temperature of the solvent for 30 min to 50 hours, yielding compounds of general formula (**I**), wherein R₁ and R₃ stand for hydrogen, R₂ stands for hydroxyl, R₄ individually stands for hydroxyl or for OCH₃ group, R₅ individually stands for C₁-C₄ alkyl group, C₂-C₄ alkenyl group, -(CH₂)ₘ-Ar, wherein Ar individually stands for phenyl or phenyl substituted with one or two groups which are selected independently from halogen or halogen alkyl, m is 0-3, X stands for oxygen or sulphur and R₆ stands for hydrogen.
which are then subjected to
b) a selective acylation of the hydroxyl group at 2'-position, preferably with acetyl group by acylation, preferably with chlorides or anhydrides of carboxylic acids with up to 4 carbon atoms, preferably with acetic acid anhydride, in the presence of inorganic or organic base, in a reaction-inert solvent at a temperature from 0-30°C, yielding 2'-O-acyl derivatives of the general formula (I), wherein R₆ stands for acetyl group and ; substituents R₁, R₂, R₃, R₄, R₅ and X have the meanings defined in a)
which are than optionally subjected to
c1) a reaction with mixed anhydrides of carboxylic acids of the formula Z-COO-R', wherein Z individually stands for hydrogen or for group Y, which is defined above, R' stands for the group which is usually used for preparation of mixed anhydrides as pivaloyl-, p-toluensulphonyl-, isobutoxycarbonyl-, ethoxycarbonyl- or isopropoxycarbonyl-group, in the presence of inorganic or organic base, in a reaction-inert solvent, preferably methylene chloride at a temperature from 0-30°C for 3-100 hours yielding compounds of the general formula (I), wherein R₁ and R₃ stand for hydrogen, R₂ stands for a group of the formula (II), wherein Y individually stands for monocyclic aromatic ring, unsubstituted or substituted with groups which are selected independently from halogen, OH, OCH₃, NO₂, NH₂, R₄ individually stands for hydroxyl or for OCH₃ group, R₅ individually stands for C₁-C₄ alkyl group, C₂-C₄ alkenyl group, -(CH₂)ₘ-Ar, wherein Ar individually stands for phenyl or phenyl substituted with one or two groups which are selected independently from halogen or halogen alkyl, m is 0-3, X stands for oxygen or sulphur and R₆ stands for acetyl group. Formed compounds are subsequently subjected to deprotection with lower alcohols, preferably in methanol, at a temperature from room temperature to the reflux temperature of the solvent, yielding a compound of the formula (**I**), wherein R₆ stands for hydrogen and all other substituents have the above meanings,
or they are optionally subjected
c2) when R₄ stands for OCH₃ group and the remaining substituents have the meanings defined in b), to oxidation of the hydroxyl group in the C-3 position of an aglycone ring according to a modified Moffat-Pfitzner process with N,N-dimethylaminopropyl-3-ethyl-carbodiimide in the presence of dimethylsulfoxide and pyridinium trifluoracetate as a catalyst in an inert organic solvent, preferably in methylene chloride, at a temperature from 10 °C to room temperature, yielding compounds of the general formula (I), wherein R₁ stands for hydrogen, R₂ together with R₃ stands for ketone, R₄ stands for OCH₃ group, R₅ individually stands for C₁-C₄ alkyl group, C₂-C₄ alkenyl group, -(CH₂)ₘ-Ar, wherein Ar individually stands for phenyl or phenyl substituted with one or two groups which are selected independently from halogen or halogen alkyl, m is 0-3, X stands for oxygen or sulphur and R₆ stand for acetyl. Formed compounds are subsequently subjected to deprotection with lower alcohols, preferably in methanol, at a temperature from room temperature to the reflux temperature of the solvent, yielding a compound of the formula (**I**), wherein R₆ stands for hydrogen and all other substituents have the above meanings,
or they are optionally subjected
c3) when R₄ stands for hydroxyl and the remaining substituents have the meanings defined in b), to oxidation described to obtain compounds of the general formula (I) from the step c2), to yield compounds with 3,6-hemiketal structure given by general formula (I), wherein R₁ stands for hydrogen, R₂ stands for hydroxyl, R₃ together with R₄ stands for ether, R₅ individually stands for C₁-C₄ alkyl group, C₂-C₄ alkenyl group, -(CH₂)ₘ-Ar, wherein Ar individually stands for phenyl or phenyl substituted with one or two groups which are selected independently from halogen or halogen alkyl, m is 0-3, X stands for oxygen or sulphur and R₆ stand for acetyl. Formed compounds are subsequently subjected to deprotection with lower alcohols, preferably in methanol, at a temperature from room temperature to the reflux temperature of the solvent, yielding a compound of the formula (I), wherein R₆ stands for hydrogen and all other substituents have the above meanings,
or they are optionally subjected,
c4) when R₄ stands for OCH₃ group and the remaining substituents have the meanings defined in b), to adequate reagents for dehydratation, preferably methylsulfonyl anhydride to transform hydroxyl group on position 3 in good leaving group, in an inert organic solvent, preferably in pyridine, at a temperature from room temperature to the reflux temperature of the solvent for 10-50 hours. Formed intermediate is subsequently subjected to reaction of elimination with adequate reagents, preferably sodium hydride, in an inert organic solvent, preferably in tetrahydrofuran, at a temperature from 10°C to room temperature, yielding 2,3-anhydro derivatives of the general formula (I), wherein R₁ together with R₂ stands for double bond, R₃ stands for hydrogen, R₄ stands for OCH₃ group, R₅ individually stands for C₁-C₄ alkyl group, C₂-C₄ alkenyl group, -(CH₂)ₘ-Ar, wherein Ar individually stands for phenyl or phenyl substituted with one or two groups which are selected independently from halogen or halogen alkyl, m is 0-3, X stands for oxygen or sulphur and R₆ stands for acetyl. Formed compounds are subsequently subjected to deprotection with lower alcohols, preferably in methanol, at a temperature from room temperature to the reflux temperature of the solvent, yielding a compound of the general formula (I), wherein R₆ stands for hydrogen and all other substituents have the above meanings
c5) when R₄ stands for hydroxyl and the remaining substituents have the meanings defined in b), to reaction of elimination with adequate reagent described in the step c4), yielding 3,6-cyclic ether of the general formula (**I**), wherein R₁ and R₂ stands for hydrogen, R₃ together with R₄ stands for ether, R₅ individually stands for C₁-C₄ alkyl group, C₂-C₄ alkenyl group, -(CH₂)ₘ-Ar, wherein:Ar individually stands for phenyl or phenyl substituted with one or two groups which are selected independently from halogen or halogen alkyl, m is 0-3, X stands for oxygen or sulphur and R₆ stands for acetyl. Formed compounds are subsequently subjected to deprotection with lower alcohols, preferably in methanol, at a temperature from room temperature to the reflux temperature of the solvent, yielding a compound of the general formula (I), wherein R₆ stands for hydrogen and all other substituents have the above meanings.

## Patentansprüche

1. Neuartige 3-Decladinosylderivate des 9a-*N*-Carbamoyl- und 9a-N-Thiocarbamoyl-9-Deoxo-9-Dihydro-9a-Aza-9a-Homoerythromycins A der allgemeinen Formel (I), 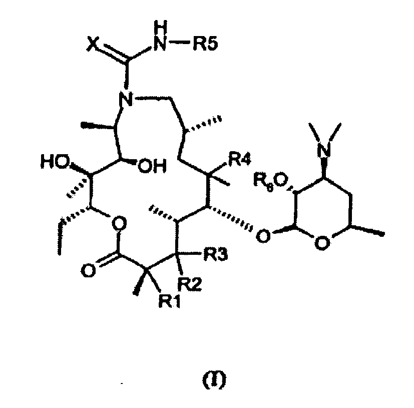 ihre pharmazeutisch annehmbaren Zusatzsalze mit anorganischen oder organischen Säuren und ihre Hydrate,
wobei
R₁ einzeln für Wasserstoff oder zusammen mit R₂ für eine Doppelbindung steht,
R₂ einzeln für Wasserstoff, Hydroxyl oder eine Gruppe der Formel (II) steht, 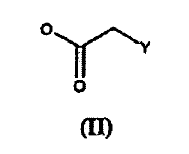 wobei
Y einzeln für einen monocyclischen aromatischen Ring steht, der unsubstituiert oder durch Gruppen, die unabhängig aus Halogen, OH, OCH₃, NO₂, NH₂ ausgewählt sind, substituiert ist,
oder
R₂ zusammen mit R₃ für Keton oder zusammen mit R₁ für eine Doppelbindung steht,
R₃ einzeln für Wasserstoff oder zusammen mit R₂ für Keton oder zusammen mit R₄ für Ether steht,
R₄ einzeln für Hydroxyl, eine OCH₃-Gruppe oder zusammen mit R₃ für Ether steht,
R₅ einzeln für eine C₁-C₄-Alkylgruppe, C₂-C₄-Alkenylgruppe, -(CH₂)ₘ-Ar steht, wobei Ar einzeln für Phenyl oder Phenyl substituiert durch eine Gruppe oder zwei Gruppen steht, die unabhängig aus Halogen oder Halogenalkyl ausgewählt sind, und m 0 bis 3 ist,
R₆ einzeln für Wasserstoff oder eine hydroxylschützende Gruppe steht,
X für Sauerstoff oder Schwefel steht.

2. Verbindung nach Anspruch 1 **dadurch gekennzeichnet, dass** R₁ und R₃ für Wasserstoff, R₂ für Hydroxyl, R₄ einzeln für Hydroxyl oder für eine OCH₃-Gruppe, R₅ einzeln für eine C₁-C₄-Alkylgruppe, C₂-C₄₋Alkenylgruppe, -(CH₂)ₘ-Ar stehen, wobei Ar einzeln für Phenyl oder Phenyl substituiert durch eine Gruppe oder zwei Gruppen steht, die unabhängig aus Halogen oder Halogenalkyl ausgewählt sind, m 0 bis 3 ist, X für Sauerstoff oder Schwefel und R₆ für Wasserstoff stehen.

3. Verbindung nach Anspruch 2 **dadurch gekennzeichnet, dass** R₄ für Hydroxyl, R₅ für eine Ethylgruppe und X für Sauerstoff stehen.

4. Verbindung nach Anspruch 2 **dadurch gekennzeichnet, dass** R₄ für Hydroxyl, R₅ für eine t-Butylgruppe und X für Sauerstoff stehen.

5. Verbindung nach Anspruch 2 **dadurch gekennzeichnet, dass** R₄ für Hydroxyl, R₅ für eine Isopropylgruppe und X für Sauerstoff stehen.

6. Verbindung nach Anspruch 2 **dadurch gekennzeichnet, dass** R₄ für eine OCH₃-Gruppe, R₅ für eine Isopropylgruppe und X für Sauerstoff stehen.

7. Verbindung nach Anspruch 2 **dadurch gekennzeichnet, dass** R₄ für Hydroxyl, R₅ für eine Allylgruppe und X für Schwefel stehen.

8. Verbindung nach Anspruch 2 **dadurch gekennzeichnet, dass** R₄ für Hydroxyl, R₅ für eine Benzylgruppe und X für Sauerstoff stehen.

9. Verbindung nach Anspruch 2 **dadurch gekennzeichnet, dass** R₄ für Hydroxyl, R₅ für eine Benzylgruppe und X für Schwefel stehen.

10. Verbindung nach Anspruch 2 **dadurch gekennzeichnet, dass** R₄ für Hydroxyl, R₅ für eine 2-Trifluormethylphenylgruppe und X für Sauerstoff stehen.

11. Verbindung nach Anspruch 2 **dadurch gekennzeichnet, dass** R₄ für Hydroxyl, R₅ für eine 3-Trifluormethylphenylgruppe und X für Sauerstoff stehen.

12. Verbindung nach Anspruch 2 **dadurch gekennzeichnet, dass** R₄ für Hydroxyl, R₅ für eine 3-Trifluormethylphenylgruppe und X für Schwefel stehen.

13. Verbindung nach Anspruch 2 **dadurch gekennzeichnet, dass** R₄ für eine OCH₃-Gruppe, R₅ für eine 2,4-Dichlorphenylgruppe und X für Sauerstoff stehen.

14. Verbindung nach Anspruch 2 **dadurch gekennzeichnet, dass** R₄ für Hydroxyl, R₅ für eine 2,4-Dichlorphenylgruppe und X für Sauerstoff stehen.

15. Verbindung nach Anspruch 1 **dadurch gekennzeichnet, dass** R₁ und R₃ für Wasserstoff, R₂ für eine Gruppe der Formel (II) stehen, wobei Y einzeln für einen monocyclischen aromatischen Ring steht, der unsubstituiert oder durch Gruppen, die unabhängig aus Halogen, OH, OCH₃, NO₂, NH₂ ausgewählt sind, substituiert ist, R₄ einzeln für Hydroxyl oder für eine OCH₃-Gruppe, R₅ einzeln für eine C₁-C₄-Alkylgruppe, C₂₋C₄-Alkenylgruppe, -(CH₂)ₘ-Ar steht, wobei Ar einzeln für Phenyl oder Phenyl substituiert durch eine Gruppe oder zwei Gruppen steht, die unabhängig aus Halogen oder Halogenalkyl ausgewählt sind, m 0 bis 3 ist, X für Sauerstoff oder Schwefel und R₆ für Wasserstoff stehen.

16. Verbindung nach Anspruch 15 **dadurch gekennzeichnet, dass** Y für 4-NO₂-substituiertes Phenyl, R₄ für Hydroxyl, R₅ für eine Isopropylgruppe und X für Sauerstoff stehen.

17. Verbindung nach Anspruch 15 **dadurch gekennzeichnet, dass** Y für 4-NO₂-substituiertes Phenyl, R₄ für Hydroxyl, R₅ für eine 2,4-Dichlorphenylgruppe und X für Sauerstoff stehen.

18. Verbindung nach Anspruch 1 **dadurch gekennzeichnet, dass** R₁, R₂ und R₆ für Wasserstoff, R₃ zusammen mit R₄ für Ether, R₅ einzeln für eine C₁₋C₄-Alkylgruppe, C₂-C₄-Alkenylgruppe, -(CH₂)ₘ-Ar stehen, wobei Ar einzeln für Phenyl oder Phenyl substituiert durch eine Gruppe oder zwei Gruppen steht, die unabhängig aus Halogen oder Halogenalkyl ausgewählt sind, m 0 bis 3 ist, X für Sauerstoff oder Schwefel steht.

19. Verbindung nach Anspruch 18 **dadurch gekennzeichnet, dass** R₅ für eine Isopropylgruppe und X für Sauerstoff stehen.

20. Verbindung nach Anspruch 18 **dadurch gekennzeichnet, dass** R₅ für eine 2,4-Dichlorphenylgruppe und X für Sauerstoff stehen.

21. Verbindung nach Anspruch 1 **dadurch gekennzeichnet, dass** R₁ und R₆ für Wasserstoff, R₂ für Hydroxyl, R₃ zusammen mit R₄ für Ether, R₅ einzeln für eine C₁-C₄-Alkylgruppe, C₂-C₄-Alkenylgruppe, -(CH₂)ₘ-Ar stehen, wobei Ar einzeln für Phenyl oder Phenyl substituiert durch eine Gruppe oder zwei Gruppen steht, die unabhängig aus Halogen oder Halogenalkyl ausgewählt sind, m 0 bis 3 ist und X für Sauerstoff oder Schwefel steht.

22. Verbindung nach Anspruch 21 **dadurch gekennzeichnet, dass** R₅ für eine Isopropylgruppe und X für Sauerstoff stehen.

23. Verbindung nach Anspruch 21 **dadurch gekennzeichnet, dass** R₅ für eine 2,4-Dichlorphenylgruppe und X für Sauerstoff stehen.

24. Verbindung nach Anspruch 1 **dadurch gekennzeichnet, dass** R₁ und R₆ für Wasserstoff, R₂ zusammen mit R₃ für Keton, R₄ für eine OCH₃₋Gruppe, R₅ einzeln für eine C₁-C₄-Alkylgruppe, C₂-C₄-Alkenylgruppe, -(CH₂)ₘ-Ar stehen, wobei Ar einzeln für Phenyl oder Phenyl substituiert durch eine Gruppe oder zwei Gruppen steht, die unabhängig aus Halogen oder Halogenalkyl ausgewählt sind, m 0 bis 3 ist und X für Sauerstoff oder Schwefel steht.

25. Verbindung nach Anspruch 24 **dadurch gekennzeichnet, dass** R₅ für eine Isopropylgruppe und X für Sauerstoff stehen.

26. Verbindung nach Anspruch 24 **dadurch gekennzeichnet, dass** R₅ für eine 2,4-Dichlorphenylgruppe und X für Sauerstoff stehen.

27. Verbindung nach Anspruch 1 **dadurch gekennzeichnet, dass** R₁ zusammen mit R₂ für eine Doppelbindung stehen, R₃ und R₆ für Wasserstoff, R₄ für eine OCH₃-Gruppe, R₅ einzeln für eine C₁-C₄₋Alkylgruppe, C₂-C₄-Alkenylgruppe, -(CH₂)ₘ-Ar stehen, wobei Ar einzeln für Phenyl oder Phenyl substituiert durch eine Gruppe oder zwei Gruppen steht, die unabhängig aus Halogen oder Halogenalkyl ausgewählt sind, m 0 bis 3 ist und X für Sauerstoff oder Schwefel steht.

28. Verbindung nach Anspruch 27 **dadurch gekennzeichnet, dass** R₅ für eine Isopropylgruppe und X für Sauerstoff stehen.

29. Verbindung nach Anspruch 27 **dadurch gekennzeichnet, dass** R₅ für eine 2,4-Dichlorphenylgruppe und X für Sauerstoff stehen.

30. Verfahren zur Zubereitung von Verbindungen der Formel (I), 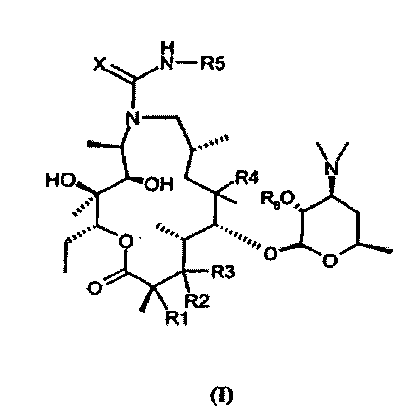 ihre pharmazeutisch annehmbaren Zusatzsalze mit anorganischen oder organischen Säuren und ihre Hydrate,
wobei
R₁ einzeln für Wasserstoff oder gemeinsam mit R₂ für eine Doppelbindung steht,
R₂ einzeln für Wasserstoff, Hydroxyl oder eine Gruppe der Formel (**II**) steht, 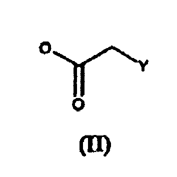 wobei
Y einzeln für einen monocyclischen aromatischen Ring steht, der unsubstituiert oder durch Gruppen, die unabhängig aus Halogen, OH, OCH₃, NO₂, NH₂ ausgewählt sind, substituiert ist,
oder
R₂ zusammen mit R₃ für Keton oder zusammen mit R₁ für eine Doppelbindung steht,
R₃ einzeln für Wasserstoff oder zusammen mit R₂ für Keton oder zusammen mit R₄ für Ether steht,
R₄ einzeln für Hydroxyl, eine OCH₃-Gruppe oder zusammen mit R₃ für Ether steht,
R₅ einzeln für eine C₁-C₄-Alkylgruppe, C₂-C₄-Alkenylgruppe, -(CH₂)ₘ-Ar steht, wobei Ar einzeln für Phenyl oder Phenyl substituiert durch eine Gruppe oder zwei Gruppen steht, die unabhängig aus Halogen oder Halogenalkyl ausgewählt sind, und m 0 bis 3 ist,
R₆ einzeln für Wasserstoff oder eine hydroxylschützende Gruppe steht,
X für Sauerstoff oder Schwefel steht, **dadurch gekennzeichnet, dass**
a) Ausgangsverbindungen der Formel 1 (Schema 1), wobei R₄ einzeln für Hydroxyl oder eine OCH₃-Gruppe steht, einer Reaktion mit Isocyanat oder Isothiocyanat der Formel R₅-N=C=X unterzogen werden, wobei R₅ und X obenstehende Bedeutungen haben, in einem reaktionsinerten Lösungsmittel, vorzugsweise Toluen oder Acetonitril, bei einer Temperatur von Raumtemperatur bis zur Refluxtemperatur des Lösungsmittels während 30 Minuten bis 50 Stunden, wodurch die Verbindungen der allgemeinen Formel (I) gebildet werden, wobei R₁ und R₃ für Wasserstoff, R₂ für Hydroxyl, R₄ einzeln für Hydroxyl oder für eine OCH₃-Gruppe, R₅ einzeln für eine C₁-C₄-Alkylgruppe, C₂-C₄₋Alkenylgruppe, -(CH₂)ₘ-Ar stehen, wobei Ar einzeln für Phenyl oder Phenyl substituiert durch eine Gruppe oder zwei Gruppen steht, die unabhängig aus Halogen oder Halogenalkyl ausgewählt sind, m 0 bis 3 ist, X für Sauerstoff oder Schwefel und R₆ für Wasserstoff stehen,
die dann
b) einer selektiven Acylierung der Hydroxylgruppe an der 2'-Position, vorzugsweise mit einer Acetylgruppe durch Acylierung vorzugsweise mit Chloriden oder Anhydriden von Carbonsäuren mit bis zu 4 C-Atomen, vorzugsweise mit Essigsäureanhydrid, in Anwesenheit einer anorganischen oder organischen Base, in einem reaktionsinerten Lösungsmittel bei einer Temperatur von 0 bis 30°C unterzogen werden, wodurch 2'-O-Acylderivate der allgemeinen Formel (**I**) gebildet werden, wobei R₆ für eine Acetylgruppe und die Substituenten R₁, R₂, R₃, R₄, R₅ und X die unter a) definierten Bedeutungen haben,
die dann optional
c1) einer Reaktion mit gemischten Anhydriden von Carbonsäuren der Formel Z-COO-R' unterzogen werden, wobei Z einzeln für Wasserstoff oder für die Gruppe Y steht, die oben definiert ist, R' für die Gruppe steht, die üblicherweise für die Zubereitung von gemischten Anhydriden wie die Pivaloyl-, p-Toluensulfonyl-, Isobutoxycarbonyl-, Ethoxycarbonyl- oder Isopropoxycarbonylgruppe, in Anwesenheit einer anorganischen oder organischen Base in einem reaktionsinerten Lösungsmittel, vorzugsweise Methylenchlorid, bei einer Temperatur von 0 bis 30°C während 3 bis 100 Stunden, wodurch Verbindungen der allgemeinen Formel (**I**) entstehen, wobei R₁ und R₃ für Wasserstoff stehen, R₂ für eine Gruppe der Formel (**II**) steht, wobei Y einzeln für einen monocyclischen aromatischen Ring steht, der unsubstituiert oder durch Gruppen substituiert ist, die unabhängig aus Halogen, OH, OCH₃, NO₂, NH₂ ausgewählt sind, R₄ einzeln für Hydroxyl oder für eine OCH₃-Gruppe steht, R₅ einzeln für eine C₁-C₄-Alkylgruppe, C₂-C₄-Alkenylgruppe, - (CH₂)ₘ-Ar steht, wobei Ar einzeln für Phenyl oder Phenyl substituiert mit einer Gruppe oder zwei Gruppen steht, die unabhängig aus Halogen oder Halogenalkyl ausgewählt sind, m 0 bis 3 ist, X für Sauerstoff oder Schwefel und R₆ für eine Acetylgruppe stehen, die gebildeten Verbindungen werden anschließend mit niedrigeren Alkoholen, vorzugsweise in Methanol, bei einer Temperatur von Raumtemperatur bis zur Refluxtemperatur des Lösungsmittels entschützt, was eine Verbindung der Formel (I) ergibt, wobei R₆ für Wasserstoff steht und alle anderen Substituenten obenstehende Bedeutungen haben,
oder sie werden optional,
c2) wenn R₄ für eine OCH₃-Gruppe steht und die übrigen Substituenten die unter b) definierte Bedeutung haben, der Oxidation der Hydroxylgruppe an der C-3 Position eines Aglyconrings nach einem modifizierten Moffat-Pfitzner-Prozess mit N,N-Dimethylaminopropyl-3-Ethylcarbodümid in Anwesenheit von Dimethylsulfoxid und Pyridiniumtrifluoracetat als Katalysator in einem inerten organischen Lösungsmittel, vorzugsweise in Methylenchlorid, bei einer Temperatur von 10 °C bis Raumtemperatur unterzogen, wodurch Verbindungen der allgemeinen Formel (I) entstehen, wobei R₁ für Wasserstoff steht, R₂ zusammen mit R₃ für Keton, R₄ für eine OCH₃-Gruppe, R₅ einzeln für eine C₁-C₄-Alkylgruppe, C₂-C₄-Alkenylgruppe, -(CH₂)ₘ-Ar stehen, wobei Ar einzeln für Phenyl oder Phenyl substituiert durch eine Gruppe oder zwei Gruppen steht, die unabhängig aus Halogen oder Halogenalkyl ausgewählt sind, m 0 bis 3 ist, X für Sauerstoff oder Schwefel und R₆ für Acetyl stehen, die gebildeten Verbindungen werden anschließend mit niedrigeren Alkoholen, vorzugsweise in Methanol, bei einer Temperatur von Raumtemperatur bis zur Refluxtemperatur des Lösungsmittels entschützt, was eine Verbindung der Formel (I) ergibt, wobei R₆ für Wasserstoff steht und alle anderen Substituenten obenstehende Bedeutungen haben,
oder sie werden optional,
c3) wenn R₄ für Hydroxyl steht und die übrigen Substituenten die unter b) definierten Bedeutungen haben, der Oxidation unterzogen, die beschrieben ist, um die Verbindungen der allgemeinen Formel (I) beim Schritt c2) zu erhalten, wobei Verbindungen mit der in der allgemeinen Formel (I) vorgegebenen 3,6-Hemiketalstruktur entstehen, wobei R₁ für Wasserstoff, R₂ für Hydroxyl, R₃ zusammen mit R₄ für Ether, R₅ einzeln für eine C₁-C₄-Alkylgruppe, C₂-C₄-Alkenylgruppe, -(CH₂)ₘ-Ar stehen, wobei Ar einzeln für Phenyl oder Phenyl substituiert durch eine Gruppe oder zwei Gruppen steht, die unabhängig aus Halogen oder Halogenalkyl ausgewählt sind, m 0 bis 3 ist, X für Sauerstoff oder Schwefel und R₆ für Acetyl stehen, die gebildeten Verbindungen werden anschließend mit niedrigeren Alkoholen, vorzugsweise in Methanol, bei einer Temperatur von Raumtemperatur bis zur Refluxtemperatur des Lösungsmittels entschützt, was eine Verbindung der Formel (**I**) ergibt, wobei R₆ für Wasserstoff steht und alle anderen Substituenten obenstehende Bedeutungen haben,
oder sie werden optional,
c4) wenn R₄ für eine OCH₃-Gruppe steht und die übrigen Substituenten die in b) definierten Bedeutungen haben, der Reaktion mit adäquaten Reagenzien für die Dehydrierung, vorzugsweise Methylsulfonylanhydrid, unterzogen, um die Hydroxylgruppe an Position 3 in eine gute Abgangsgruppe in einem inerten organischen Lösungsmittel, vorzugsweise in Pyridin, bei einer Temperatur von Raumtemperatur bis zur Refluxtemperatur des Lösungsmittels während 10 bis 50 Stunden umzuwandeln, das gebildete Zwischenprodukt wird anschließend der Eliminierungsreaktion mit adäquaten Reagenzien, vorzugsweise Natriumhydrid, in einem inerten organischen Lösungsmittel, vorzugsweise in Tetrahydrofuran, bei einer Temperatur von 10 °C bis Raumtemperatur unterzogen, was 2,3-Anhydroderivate der allgemeinen Formel (I) ergibt, wobei R₁ zusammen mit R₂ für eine Doppelbindung, R₃ für Wasserstoff, R₄ für eine OCH₃-Gruppe, R₅ einzeln für eine C₁-C₄₋Alkylgruppe, C₂-C₄-Alkenylgruppe, -(CH₂)ₘ-Ar stehen, wobei Ar einzeln für Phenyl oder Phenyl substituiert mit einer Gruppe oder zwei Gruppen steht, die unabhängig aus Halogen oder Halogenalkyl ausgewählt sind, m 0 bis 3 ist, X für Sauerstoff oder Schwefel und R₆ für Acetyl stehen, die gebildeten Verbindungen werden anschließend mit niedrigeren Alkoholen, vorzugsweise in Methanol, bei einer Temperatur von Raumtemperatur bis zur Refluxtemperatur des Lösungsmittels entschützt, was eine Verbindung der allgemeinen Formel (I) ergibt, wobei R₆ für Wasserstoff steht und alle anderen Substituenten obenstehende Bedeutungen haben,
c5) wenn R₄ für Hydroxyl steht und die übrigen Substituenten die in b) definierten Bedeutungen haben, der im Schritt c4) beschriebenen Eliminierungsreaktion mit adäquaten Reagenzien unterzogen, wodurch ein 3,6-cyclischer Ether der allgemeinen Formel (I) gebildet wird, wobei R₁ und R₂ für Wasserstoff, R₃ zusammen mit R₄ für Ether, R₅ einzeln für eine C₁-C₄-Alkylgruppe, C₂-C₄-Alkenylgruppe, -(CH₂)ₘ-Ar stehen, wobei Ar einzeln für Phenyl oder Phenyl substituiert mit einer Gruppe oder zwei Gruppen steht, die unabhängig aus Halogen oder Halogenalkyl ausgewählt sind, m 0 bis 3 ist, X für Sauerstoff oder Schwefel und R₆ für Acetyl stehen, die gebildeten Verbindungen werden anschließend mit niedrigeren Alkoholen, vorzugsweise in Methanol, bei einer Temperatur von Raumtemperatur bis zur Refluxtemperatur des Lösungsmittels entschützt, was eine Verbindung der Formel (I) ergibt, wobei R₆ für Wasserstoff steht und alle anderen Substituenten obenstehende Bedeutungen haben.

## Revendications

1. Nouveaux dérivés 3-décladinosyle de la 9a-N-carbamoyl-9-désoxo-9-dihydro-9a-aza-9a-homoérythromycine et de la 9a-N-thiocarbamoyl-9-désoxo-9-dihydro-9a-aza-9a-homoérythromycine A de formule générale (I), 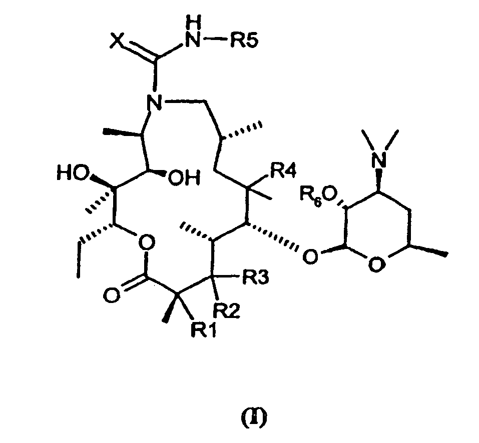 leurs sels d'addition pharmaceutiquement acceptables avec des acides inorganiques ou organiques et leurs hydrates, où
R₁ représente individuellement de l'hydrogène ou, avec R₂, une double liaison,
R₂ représente individuellement de l'hydrogène, hydroxyle ou un groupe de formule (II), 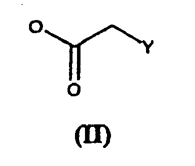 où
Y représente individuellement un cycle aromatique monocyclique, non substitué ou substitué par des groupes qui sont choisis indépendamment parmi halogène, OH, OCH₃, NO₂, NH₂
ou
R₂ représente, avec R₃, cétone ou, avec R₁, une double liaison,
R₃ représente individuellement hydrogène ou, avec R₂, cétone ou, avec R₄, éther,
R₄ représente individuellement hydroxyle, un groupe OCH₃ ou, avec R₃, éther,
R₅ représente individuellement un groupe alkyle en C₁-C₄, un groupe alcényle en C₂-C₄, -(CH₂)ₘ-Ar, où Ar représente individuellement phényle ou phényle substitué par un ou deux groupes qui sont choisis indépendamment parmi halogène ou halogénoalkyle, et m vaut 0-3,
R₆ représente individuellement hydrogène ou un groupe protégeant l'hydroxyle,
X représente de l'oxygène ou du soufre.

2. Composé selon la revendication 1, **caractérisé en ce que** R₁ et R₃ représentent hydrogène, R₂ représente hydroxyle, R₄ représente individuellement hydroxyle ou un groupe OCH₃, R₅ représente individuellement un groupe alkyle en C₁-C₄, un groupe alcényle en C₂-C₄, -(CH₂)ₘ-Ar, où Ar représente individuellement phényle ou phényle substitué par un ou deux groupes qui sont choisis indépendamment parmi halogène ou halogénoalkyle, m vaut 0-3, X représente oxygène ou soufre et R₆ représente hydrogène.

3. Composé selon la revendication 2, **caractérisé en ce que** R₄ représente hydroxyle, R₅ représente un groupe éthyle et X représente oxygène.

4. Composé selon la revendication 2, **caractérisé en ce que** R₄ représente hydroxyle, R₅ représente un groupe t-butyle et X représente oxygène.

5. Composé selon la revendication 2, **caractérisé en ce que** R₄ représente hydroxyle, R₅ représente un groupe isopropyle et X représente oxygène.

6. Composé selon la revendication 2, **caractérisé en ce que** R₄ représente un groupe OCH₃, R₅ représente un groupe isopropyle et X représente oxygène.

7. Composé selon la revendication 2, **caractérisé en ce que** R₄ représente hydroxyle, R₅ représente un groupe allyle et X représente soufre.

8. Composé selon la revendication 2, **caractérisé en ce que** R₄ représente hydroxyle, R₅ représente un groupe benzyle et X représente oxygène.

9. Composé selon la revendication 2, **caractérisé en ce que** R₄ représente hydroxyle, R₅ représente un groupe benzyle et X représente soufre.

10. Composé selon la revendication 2, **caractérisé en ce que** R₄ représente hydroxyle, R₅ représente un groupe 2-trifluorométhylphényle et X représente oxygène.

11. Composé selon la revendication 2, **caractérisé en ce que** R₄ représente hydroxyle, R₅ représente un groupe 3-trifluorométhylphényle et X représente oxygène.

12. Composé selon la revendication 2, **caractérisé en ce que** R₄ représente hydroxyle, R₅ représente un groupe 3-trifluorométhylphényle et X représente soufre.

13. Composé selon la revendication 2, **caractérisé en ce que** R₄ représente un groupe OCH₃, R₅ représente un groupe 2,4-dichlorophényle et X représente oxygène.

14. Composé selon la revendication 2, **caractérisé en ce que** R₄ représente hydroxyle, R₅ représente un groupe 2,4-dichlorophényle et X représente oxygène.

15. Composé selon la revendication 1, **caractérisé en ce que** R₁ et R₃ représentent hydrogène, R₂ représente un groupe de formule (II), où Y représente individuellement un cycle aromatique monocyclique, non substitué ou substitué par des groupes qui sont choisis indépendamment parmi halogène, OH, OCH₃, NO₂, NH₂, R₄ représente individuellement hydroxyle ou un groupe OCH₃, R₅ représente individuellement un groupe alkyle en C₁-C₄, un groupe alcényle en C₂-C₄, -(CH₂)ₘ-Ar, où Ar représente individuellement phényle ou phényle substitué par un ou deux groupes qui sont choisis indépendamment parmi halogène ou halogénoalkyle, m vaut 0-3, X représente oxygène ou soufre et R₆ représente hydrogène.

16. Composé selon la revendication 15, **caractérisé en ce que** Y représente phényle substitué par 4-NO₂, R₄ représente hydroxyle, R₅ représente un groupe isopropyle et X représente oxygène.

17. Composé selon la revendication 15, **caractérisé en ce que** Y représente phényle substitué par 4-NO₂, R₄ représente hydroxyle, R₅ représente un groupe 2,4-dichlorophényle et X représente oxygène.

18. Composé selon la revendication 1, **caractérisé en ce que** R₁, R₂ et R₆ représentent hydrogène, R₃ avec R₄ représente éther, R₅ représente individuellement un groupe alkyle en C₁-C₄, un groupe alcényle en C₂-C₄, -(CH₂)ₘ-Ar, où Ar représente individuellement phényle ou phényle substitué par un ou deux groupes qui sont choisis indépendamment parmi halogène ou halogénoalkyle, m vaut 0-3, X représente oxygène ou soufre.

19. Composé selon la revendication 18, **caractérisé en ce que** R₅ représente un groupe isopropyle et X représente oxygène.

20. Composé selon la revendication 18, **caractérisé en ce que** R₅ représente un groupe 2,4-dichlorophényle et X représente oxygène.

21. Composé selon la revendication 1, **caractérisé en ce que** R₁ et R₆ représentent hydrogène, R₂ représente hydroxyle, R₃ avec R₄ représente éther, R₅ représente individuellement un groupe alkyle en C₁-C₄, un groupe alcényle en C₂-C₄, -(CH₂)ₘ-Ar, où Ar représente individuellement phényle ou phényle substitué par un ou deux groupes qui sont choisis indépendamment parmi halogène ou halogénoalkyle, m vaut 0-3 et X représente oxygène ou soufre.

22. Composé selon la revendication 21, **caractérisé en ce que** R₅ représente un groupe isopropyle et X représente oxygène.

23. Composé selon la revendication 21, **caractérisé en ce que** R₅ représente un groupe 2,4-dichlorophényle et X représente oxygène.

24. Composé selon la revendication 1, **caractérisé en ce que** R₁ et R₆ représentent hydrogène, R₂ avec R₃ représente cétone, R₄ représente un groupe OCH₃, R₅ représente individuellement un groupe alkyle en C₁-C₄, un groupe alcényle en C₂-C₄, -(CH₂)ₘ-Ar, où Ar représente individuellement phényle ou phényle substitué par un ou deux groupes qui sont choisis indépendamment parmi halogène ou halogénoalkyle, m vaut 0-3 et X représente oxygène ou soufre.

25. Composé selon la revendication 24, **caractérisé en ce que** R₅ représente un groupe isopropyle et X représente oxygène.

26. Composé selon la revendication 24, **caractérisé en ce que** R₅ représente un groupe 2,4-dichlorophényle et X représente oxygène.

27. Composé selon la revendication 1, **caractérisé en ce que** R₁ avec R₂ représente une double liaison, R₃ et R₆ représentent hydrogène, R₄ représente un groupe OCH₃, R₅ représente individuellement un groupe alkyle en C₁-C₄, un groupe alcényle en C₂-C₄, -(CH₂)ₘ-Ar, où Ar représente individuellement phényle ou phényle substitué par un ou deux groupes qui sont choisis indépendamment parmi halogène ou halogénoalkyle, m vaut 0-3 et X représente oxygène ou soufre.

28. Composé selon la revendication 27, **caractérisé en ce que** R₅ représente un groupe isopropyle et X représente oxygène.

29. Composé selon la revendication 27, **caractérisé en ce que** R₅ représente un groupe 2,4-dichlorophényle et X représente oxygène.

30. Procédé de préparation de composés de formule (I), 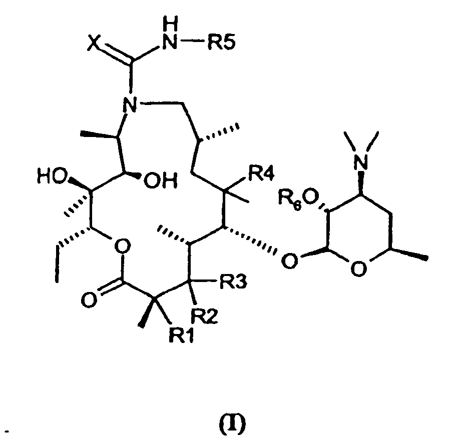 leurs sels d'addition pharmaceutiquement acceptables avec des acides inorganiques ou organiques et leurs hydrates,
où
R₁ représente individuellement hydrogène ou, avec R₂, une double liaison,
R₂ représente individuellement hydrogène, hydroxyle ou un groupe de formule (II), 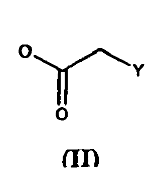 où
Y représente individuellement un cycle aromatique monocyclique, non substitué ou substitué par des groupes qui sont choisis indépendamment parmi halogène, OH, OCH₃, NO₂, NH₂ ou
R₂ avec R₃ représente cétone ou, avec R₁, une double liaison,
R₃ représente individuellement hydrogène ou, avec R₂, cétone ou, avec R₄, éther,
R₄ représente individuellement hydroxyle, un groupe OCH₃ ou, avec R₃, éther,
R₅ représente individuellement un groupe alkyle en C₁-C₄, un groupe alcényle en C₂-C₄, -(CH₂)ₘ-Ar, où Ar représente individuellement phényle ou phényle substitué par un ou deux groupes qui sont choisis indépendamment parmi halogène ou halogénoalkyle et m vaut 0-3,
R₆ représente individuellement hydrogène ou un groupe protégeant l'hydroxyle,
X représente oxygène ou soufre, **caractérisé en ce que**
a) des composés de départ de formule 1 (schéma 1), où R₄ représente individuellement hydroxyle ou un groupe OCH₃, sont soumis à une réaction avec un isocyanate ou un isothiocyanate de formule R₅-N=C=X où R₅ et X ont les significations ci-dessus, dans un solvant inerte à la réaction, de préférence le toluène ou l'acétonitrile, à une température allant de la température ambiante à la température de reflux du solvant pendant 30 min à 50 heures, ce qui permet d'obtenir des composés de formule générale (I), où R₁ et R₃ représentent hydrogène, R₂ représente hydroxyle, R₄ représente individuellement hydroxyle ou un groupe OCH₃, R₅ représente individuellement un groupe alkyle en C₁-C₄, un groupe alcényle en C₂-C₄, -(CH₂)ₘ-Ar, où Ar représente individuellement phényle ou phényle substitué par un ou deux groupes qui sont choisis indépendamment parmi halogène ou halogénoalkyle, m vaut 0-3, X représente oxygène ou soufre et R₆ représente hydrogène,
qui sont ensuite soumis à
b) une acylation sélective du groupe hydroxyle en position 2', de préférence avec un groupe acétyle par acylation, de préférence avec des chlorures ou des anhydrides d'acides carboxyliques comprenant jusqu'à 4 atomes de carbone, de préférence avec un anhydride de l'acide acétique, en présente d'une base inorganique ou organique, dans un solvant inerte à la réaction à une température de 0-30°C, ce qui permet d'obtenir des dérivés 2'-O-acyle de formule générale (I), où R₆ représente un groupe acétyle et les substituants R₁, R₂, R₃, R₄, R₅ et X ont les significations définies en a)
qui sont alors éventuellement soumis à
c1) une réaction avec des anhydrides mixtes d'acides carboxyliques de formule Z-COO-R', où Z représente individuellement hydrogène ou un groupe Y, qui est défini ci-dessus, R' représente le groupe qui est usuellement utilisé pour la préparation d'anhydrides mixtes tels qu'un groupe pivaloyle, p-toluènesulfonyle, isobutoxycarbonyle, éthoxycarbonyle ou isopropoxycarbonyle, en présence d'une base inorganique ou organique, dans un solvant inerte à la réaction, de préférence le chlorure de méthylène, à une température de 0-30°C pendant 3-100 heures ce qui permet d'obtenir des composés de formule générale (I), où R₁ et R₃ représentent hydrogène, R₂ représente un groupe de formule (II), où Y représente individuellement un cycle aromatique monocyclique, non substitué ou substitué par des groupes qui sont choisis indépendamment parmi halogène, OH, OCH₃, NO₂, NH₂, R₄ représente individuellement hydroxyle ou un groupe OCH₃, R₅ représente individuellement un groupe alkyle en C₁-C₄, un groupe alcényle en C₂-C₄, -(CH₂)ₘ-Ar, où Ar représente individuellement phényle ou phényle substitué par un ou deux groupes qui sont choisis indépendamment parmi halogène ou halogénoalkyle, m vaut 0-3, X représente oxygène ou soufre et R₆ représente un groupe acétyle, les composés formés sont ensuite soumis à une déprotection avec des alcools inférieurs, de préférence dans du méthanol, à une température allant de la température ambiante à la température de reflux du solvant, ce qui permet d'obtenir un composé de formule (I), où R₆ représente hydrogène et tous les autres substituants ont les significations ci-dessus,
ou ils sont éventuellement soumis à
c2) lorsque R₄ représente un groupe OCH₃ et les autres substituants ont les significations définies au point b), à l'oxydation du groupe hydroxyle en position C-3 d'un cycle aglycone conformément à un procédé Moffat-Pfitzner modifié avec du N,N-diméthylaminopropyl-3-éthyl-carbodiimide en présence de diméthylsulfoxyde et de trifluoroacétate de pyridinium comme catalyseur dans un solvant organique inerte, de préférence dans du chlorure de méthylène, à une température allant de 10°C à la température ambiante, ce qui permet d'obtenir des composés de formule générale (I), où R₁ représente hydrogène, R₂ avec R₃ représente cétone, R₄ représente un groupe OCH₃, R₅ représente individuellement un groupe alkyle en C₁-C₄, un groupe alcényle en C₂-C₄, -(CH₂)ₘ-Ar, où Ar représente individuellement phényle ou phényle substitué par un ou deux groupes qui sont choisis indépendamment parmi halogène ou halogénoalkyle, m vaut 0-3, X représente oxygène ou soufre et R₆ représente acétyle, les composés formés sont ensuite soumis à une déprotection avec des alcools inférieurs, de préférence dans du méthanol, à une température allant de la température ambiante à la température de reflux du solvant, ce qui permet d'obtenir un composé de formule (I), où R₆ représente hydrogène et tous les autres substituants ont les significations ci-dessus,
ou ils sont éventuellement soumis à
c3) lorsque R₄ représente hydroxyle et les autres substituants ont les significations définies au point b), à l'oxydation décrite pour obtenir des composés de formule générale (I) de l'étape c2), ce qui permet d'obtenir des composés avec une structure 3,6-hémicétal donnée par la formule générale (I), où R₁ représente hydrogène, R₂ représente hydroxyle, R₃ avec R₄ représente éther, R₅ représente individuellement un groupe alkyle en C₁-C₄, un groupe alcényle en C₂-C₄, -(CH₂)ₘ-Ar, où Ar représente individuellement phényle ou phényle substitué par un ou deux groupes qui sont choisis indépendamment parmi halogène ou halogénoalkyle, m vaut 0-3, X représente oxygène ou soufre et R₆ représente acétyle, les composés formés sont ensuite soumis à une déprotection avec des alcools inférieurs, de préférence dans du méthanol, à une température allant de la température ambiante à la température de reflux du solvant, ce qui permet d'obtenir un composé de formule (I), où R₆ représente hydrogène et tous les autres substituants ont les significations ci-dessus,
ou ils sont éventuellement soumis à
c4) lorsque R₄ représente un groupe OCH₃ et les autres substituants présentent les significations définies au point b), à des réactifs adéquats pour une déshydratation, de préférence le méthylsulfonylanhydride pour transformer le groupe hydroxyle en 3ème position en un bon groupe sortant, dans un solvant organique inerte, de préférence dans la pyridine, à une température allant de la température ambiante à la température de reflux du solvant pendant 10-50 heures, l'intermédiaire formé est ensuite soumis à une réaction d'élimination avec des réactifs adéquats, de préférence l'hydrure de sodium, dans un solvant organique inerte, de préférence dans le tétrahydrofuranne, à une température allant de 10°C à la température ambiante, ce qui permet d'obtenir des dérivés 2,3-anhydro de formule générale (I), où R₁ avec R₂ représente une double liaison, R₃ représente hydrogène, R₄ représente un groupe OCH₃, R₅ représente individuellement un groupe alkyle en C₁-C₄, un groupe alcényle en C₂-C₄, -(CH₂)ₘ-Ar, où Ar représente individuellement phényle ou phényle substitué par un ou deux groupes qui sont choisis indépendamment parmi halogène ou halogénoalkyle, m vaut 0-3, X représente oxygène ou soufre et R₆ représente acétyle, les composés formés sont ensuite soumis à une déprotection avec des alcools inférieurs, de préférence dans du méthanol, à une température allant de la température ambiante à la température de reflux du solvant, ce qui permet d'obtenir un composé de formule générale (I), où R₆ représente hydrogène et tous les autres substituants ont les significations ci-dessus,
c5) lorsque R₄ représente hydroxyle et les autres substituants ont les significations définies au point b), à une réaction d'élimination avec un réactif adéquat décrit dans l'étape c4), ce qui permet d'obtenir un éther cyclique 3,6 de formule générale (I), où R₁ et R₂ représentent hydrogène, R₃ avec R₄ représente éther, R₅ représente individuellement un groupe alkyle en C₁-C₄, un groupe alcényle en C₂-C₄, -(CH₂)ₘ-Ar, où Ar représente individuellement phényle ou phényle substitué par un ou deux groupes qui sont choisis indépendamment parmi halogène ou halogénoalkyle, m vaut 0-3, X représente oxygène ou soufre et R₆ représente acétyle. Les composés formés sont ensuite soumis à une déprotection avec des alcools inférieurs, de préférence dans du méthanol, à une température allant de la température ambiante à la température de reflux du solvant, ce qui permet d'obtenir un composé de formule (I), où R₆ représente hydrogène et tous les autres substituants ont les significations ci-dessus,
